# EUROPEAN PATENT SPECIFICATION

(11) **EP 3 311 797 B1**
(45) Date of publication and mention of the grant of the patent: **26.02.2020**
(21) Application number: 16814213.1
(22) Date of filing: 13.06.2016
(51) Int. Cl.: A61K 8/64, A61K 8/34, A61K 8/36, A61K 8/86, A61K 8/90, A61K 8/91, A61Q 19/00

(54) **STICK-SHAPED SUBSTRATE CONTAINING LIPID-PEPTIDE-TYPE COMPOUND**
STIFTFÖRMIGES SUBSTRAT MIT LIPID-PEPTID-VERBINDUNG
MATÉRIAU DE BASE EN BÂTONNET CONTENANT UN COMPOSÉ PEPTIDE LIPIDIQUE

(30) Priority: 24.06.2015 JP 2015127108
(43) Date of publication of application: 25.04.2018
(73) Proprietor: Nissan Chemical Corporation, Tokyo (JP)
(72) Inventor: KASHINO, Tsubasa, Funabashi-shi Chiba 274-8507 (JP); IMOTO, Takayuki, Funabashi-shi Chiba 274-8507 (JP)
(74) Representative: Hoffmann Eitle
(86) International application number: PCT/JP2016/067551
(87) International publication number: WO 2016/208442

(56) References cited:
- EP-A1- 2 865 442
- EP-A1- 3 311 798
- WO-A1-2011/052613
- WO-A1-2014/005402
- WO-A1-2015/005219
- WO-A1-2015/099074
- JP-A- 2000 511 159
- KR-A- 20110 030 802

## Description

### TECHNICAL FIELD

The present invention relates to a solid base material for external use on skin comprising a lipid peptide compound, which is preferably a stick-shaped base material, and an aqueous composition comprising a lipid peptide compound that is useful as a premix raw material for the solid base material for external use on skin.

### BACKGROUND ART

Aqueous solid compositions have been marketed or proposed as various products for cosmetics and the like, because they give a highly refreshing feel upon application to the skin or the like, and give a light feel upon use without leaving stickiness after use, as compared to oleaginous solid compositions.

Conventionally proposed aqueous solid compositions include an oil-in-water solid makeup cosmetic preparation comprising water, a fatty acid soap, an oil, and powder (Patent Document 1); and a stick-shaped aqueous cosmetic preparation comprising an alkyl and/or alkenyl oligoglycoside, an oily substance, and a nonionic emulsifier (Patent Document 2).

Aqueous gel compositions are also one example of such aqueous solid compositions. Various compounds such as a polymer gelator and a low-molecular-weight gelator have been proposed as additives for producing such aqueous gels. For example, a low-molecular-weight lipid peptide gelator that has high biological safety and is expected to be applied to pharmaceutical materials or the like has been recently proposed. Examples of such gelator systems can be found in EP 2 865 442 A1, EP 3 311 798 A1, WO 2011/052613 A1, WO 2014/0054702 A1 and WO 2015/005219 A1.

### Prior Art Documents

### Patent Documents

Patent Document 1: Japanese Patent Application Publication No. H3-279319 (JP H3-279319 A)
Patent Document 2: Japanese Patent Application Publication (Translation of PCT Application) No. 2002-516818 (JP 2002-516818 A)

### SUMMARY OF THE INVENTION

### Problem to be Solved by the Invention

An aqueous gel obtained using the above-mentioned low-molecular-weight lipid peptide gelator has a relatively low breaking strength, which makes it difficult to apply the aqueous gel to products for applications requiring a certain level of strength, such as a stick-shaped solid base material for external use on skin.

In view of the above-described circumstances, a problem to be solved by the present invention is to provide a solid base material for external use on skin that has a breaking strength sufficiently high to be usable as a stick-shaped base material or the like.

### Means for Solving the Problem

As a result of diligent study to solve the aforementioned problem, the present inventors have found that, in the formation of a hydrogel using water and a lipid peptide compound (gelator) comprising a low-molecular-weight lipid peptide or a pharmaceutically usable salt thereof, a 1,3-alkanediol serving as a solubilizer for the lipid peptide compound and a surfactant can be added to produce a gel having a breaking strength dramatically higher than that of conventional gels, which can be suitably used as a solid base material for external use on skin, particularly a stick-shaped base material, thus completing the present invention.

In summary, a first aspect of the present invention relates to a solid base **material for external use on skin comprising:**
a surfactant being one or more compounds selected from the group consisting of ethylene glycol alkyl ethers, phospholipids, polyglycerin fatty acid esters and polyoxyethylene polyoxypropylene alkyl ethers;
water; a 2-ethyl-1,3-hexanediol; and
a lipid peptide compound comprising at least one of compounds of formulas (1) to (3): 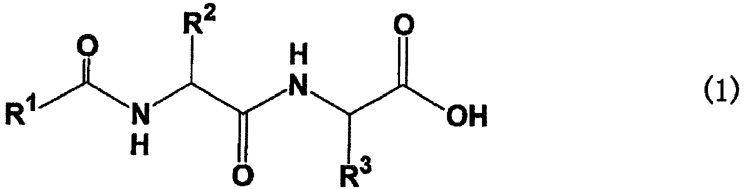
(wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); 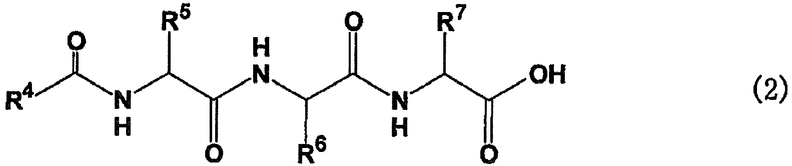 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); 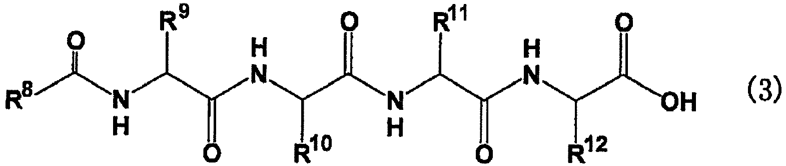 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); or
pharmacologically usable salts thereof.

A second aspect of the present invention relates to the solid base material for external use on skin according to the first aspect, which further comprises at least one fatty acid.

A third aspect of the present invention relates to the solid base material for external use on skin according to the first or second aspect, which further comprises at least one oleaginous base.

A fourth aspect of the present invention relates to the solid base material for external use on skin according to any one of the first to third aspects, which further comprises at least one organic acid different from the fatty acid.

A fifth aspect of the present invention relates to the solid base material for external use on skin according to any one of the first to fourth aspects, which further comprises at least one polyhydric alcohol different from the 2-ethyl-1,3-hexanediol.

A sixth aspect of the present invention relates to the solid base material for external use on skin according to any one of the first to fifth aspects, which further comprises at least one polymeric emulsifier.

A seventh aspect of the present invention relates to the solid base material for external use on skin according to any one of the first to sixth aspects, wherein the surfactant is one or more compounds selected from the group consisting of ethylene glycol alkyl ethers, phospholipids, polyglycerin fatty acid esters, and polyoxyethylene polyoxypropylene alkyl ethers.

An eighth aspect of the present invention relates to the solid base material for external use on skin according to any one of the second to seventh aspects, wherein the fatty acid is stearic acid.

A ninth aspect of the present invention relates to the solid base material for external use on skin according to any one of the fifth to eighth aspects, wherein the polyhydric alcohol is glycerin, propylene glycol, or polyethylene glycol.

A tenth aspect of the present invention relates to the solid base material for external use on skin according to any one of the sixth to ninth aspects, wherein the polymeric emulsifier is at least one polymer compound selected from the group consisting of graft polymer compounds each having a hydrophilic main chain and a hydrophobic moiety grafted thereto and block polymer compounds each composed of hydrophobic and hydrophilic structural units.

An eleventh aspect of the present invention relates to the solid base material for external use on skin according to any one of the first to tenth aspects, which is used for cosmetics or pharmaceuticals.

A twelfth aspect of the present invention relates to the solid base material for external use on skin according to any one of the first to eleventh aspects, which is stick-shaped.

A thirteenth aspect of the present invention relates to an aqueous composition comprising a surfactant, a surfactant being one or more compounds selected from ethylene glycol alkyl ethers, phospholipids, polyglycerin fatty acid esters and polyoxyethylene polyoxypropylene alkyl ethers, water, a 2-ethyl-1,3-hexanediol, and a lipid peptide compound comprising at least one of compounds of formulas (1) to (3) or pharmacologically usable salts thereof.

A fourteenth aspect of the present invention relates to the aqueous composition according to the thirteenth aspects, which further comprises at least one fatty acid.

A fifteenth aspect of the present invention relates to the aqueous composition according to the thirteenth or fourteenth aspect, which further comprises at least one polyhydric alcohol different from the 2-ethyl-1,3-hexanediol.

A sixteenth aspect of the present invention relates to the aqueous composition according to any one of the thirteenth to fifteenth aspects, wherein the surfactant is one or more compounds selected from the group consisting of ethylene glycol alkyl ethers, phospholipids, polyglycerin fatty acid esters, and polyoxyethylene polyoxypropylene alkyl ethers.

A seventeenth aspect of the present invention relates to the aqueous composition according to any one of the thirteenth to sixteenth aspects, which is a premix for preparing a solid base material for external use on skin.

An eighteenth aspect of the present invention relates to a method for producing the solid base material for external use on skin according to the first aspect, comprising:
a heating step of heating the aqueous composition according to any one of the thirteenth to seventeenth aspects to a temperature not lower than room temperature and lower than 100°C;
a mixing step of adding the heated aqueous composition to an aqueous phase heated to a temperature not lower than room temperature and lower than 100°C, followed by mixing, or adding an aqueous phase heated to a temperature not lower than room temperature and lower than 100°C to the heated aqueous composition, followed by mixing; and
a cooling step of cooling the resulting mixture to form a gel.

A nineteenth aspect of the present invention relates to the method according to the eighteenth aspect, wherein the aqueous phase comprises at least one oleaginous base.

A twentieth aspect of the present invention relates to the method according to the eighteenth or nineteenth aspect, wherein the aqueous phase comprises at least one polyhydric alcohol different from the 2-ethyl-1,3-hexanediol.

A twenty-first aspect of the present invention relates to the method according to the eighteenth aspect, wherein the aqueous phase comprises at least one polymeric emulsifier.

### Effects of the Invention

The present invention provides a solid base material for external use on skin having a strength higher than that of conventional aqueous gel base materials.

The present invention also provides a solid base material for external use on skin, which is a gel base material that can exhibit a high water content of approximately 90% by mass, and can contain an oleaginous ingredient simultaneously.

The lipid peptide compound contained in the solid base material for external use on skin of the present invention is a highly safe artificial low-molecular-weight compound that is composed of a lipid and a peptide only. Moreover, for example, the lipid peptide compound allows an aqueous gel to be formed without using a crosslinking agent or the like that is required to form a conventionally proposed synthetic polymer gel. As a result, no unreacted matter such as unreacted crosslinking agent remains in the resulting solid base material for external use on skin.

Furthermore, the main ingredients contained as additives in the solid base material for external use on skin of the present invention are versatile additives for foodstuffs, cosmetics, and pharmaceuticals.

That is, the solid base material for external use on skin of the present invention has a high level of biological safety, and hence, is extremely useful for the above-described uses, particularly from the viewpoint of a high level of safety required in materials for medicinal use or materials for cosmetic use, for example.

Furthermore, the solid base material for external use on skin of the present invention is expected to serve as a base material that gives a refreshing feel, is neither broken nor deformed, and spreads well when applied to human skin or the like, and thus, is extremely useful as a base material for cosmetics or pharmaceuticals, and particularly as a stick-shaped base material.

The present invention also provides an aqueous composition suitable as a premix raw material for the solid base material for external use on skin.

Through the use of the aqueous composition, the present invention provides a solid base material for external use on skin in the form of a gel that has a strength required for a stick-shaped base material, particularly even when it contains a large amount of an organic acid such as ascorbic acid.

### MODES FOR CARRYING OUT THE INVENTION

The present invention relates to a solid base material for external use on skin comprising a a surfactant being one or more compounds selected from ethylene glycol alkyl ethers, phospholipids, polyglycerin fatty acid esters and polyoxyethylene polyoxypropylene alkyl ethers, water, a 2-ethyl-1,3-hexanediol, and a lipid peptide compound comprising at least one of compounds of formulas (1) to (3) or pharmacologically usable salts thereof, the solid base material further optionally comprising a 1,2-alkanediol or glycerin, a fatty acid, an oleaginous base, an organic acid, a polyhydric alcohol, a polymeric emulsifier, and other additives.

The present invention also relates to an aqueous composition comprising the a surfactant being one or more compounds selected from ethylene glycol alkyl ethers, phospholipids, polyglycerin fatty acid esters and polyoxyethylene polyoxypropylene alkyl ethers, water, a 2-ethyl-1,3-hexanediol, and the lipid peptide compound, the aqueous composition further optionally comprising a fatty acid and a polyhydric alcohol, as well as an oleaginous base, an organic acid, a polymeric emulsifier, and other additives.

The solid base material for external use on skin of the present invention is suitable for cosmetics or pharmaceuticals, and can be used particularly as a stick-shaped base material. As used herein, the "stick-shaped base material" refers to a bar-shaped base material having a strength sufficiently high to maintain the bar shape and to be applicable to the skin or the like (i.e., capable of maintaining the shape upon application). The strength required in the stick-shaped base material is, for example, a breaking strength of 0.4 × 10⁵ to 8.0 × 10⁵ Pa, preferably 1.0 × 10⁵ to 7.0 × 10⁵ Pa, and more preferably 1.0 × 10⁵ to 6.0 × 10⁵ Pa.

Each of the components will be described hereinafter.

### [Lipid Peptide Compound]

As the lipid peptide compound to be used in the solid base material for external use on skin or the aqueous composition of the present invention, the compounds (lipid peptides) of formulas (1) to (3) or pharmacologically usable salts thereof (low-molecular-weight compounds each having a lipid moiety as a hydrophobic moiety and a peptide moiety as a hydrophilic moiety) can be used.

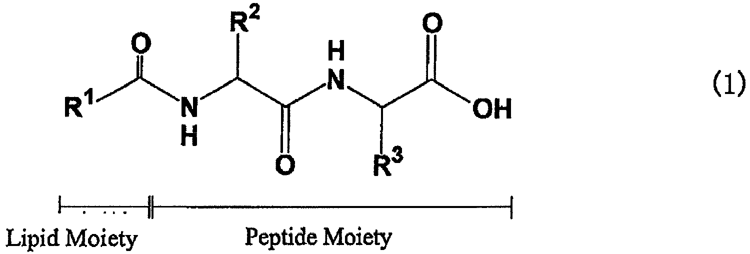

In formula (1), R¹ is a C₉₋₂₃ aliphatic group, and preferably a linear aliphatic group having a carbon atom number of 11 to 23, and optionally having zero to two unsaturated bonds.

Specific examples of the lipid moiety (acyl group) composed of R¹ and the adjacent carbonyl group include lauroyl group, dodecylcarbonyl group, myristoyl group, tetradecylcarbonyl group, palmitoyl group, margaroyl group, oleoyl group, elaidoyl group, linoleoyl group, stearoyl group, vaccenoyl group, octadecylcarbonyl group, arachidoyl group, eicosylcarbonyl group, behenoyl group, elkanoyl group, docosylcarbonyl group, lignoceroyl group, and nervonoyl group; and particularly preferred examples include lauroyl group, myristoyl group, palmitoyl group, margaroyl group, stearoyl group, oleoyl group, elaidoyl group, and behenoyl group.

In formula (1), R² included in the peptide moiety is a hydrogen atom or a C₁₋₄ alkyl group optionally having a C₁ or C₂ branched chain.

The C₁₋₄ alkyl group optionally having a C₁ or C₂ branched chain refers to an alkyl group that has a C₁₋₄ main chain, and optionally has a C₁ or C₂ branched chain. Specific examples of the C₁₋₄ alkyl group include methyl group, ethyl group, n-propyl group, i-propyl group, n-butyl group, i-butyl group, sec-butyl group, and tert-butyl group.

R² is preferably a hydrogen atom or a C₁₋₃ alkyl group optionally having a C₁ branched chain, and is more preferably a hydrogen atom.

The C₁₋₃ alkyl group optionally having a C₁ branched chain refers to an alkyl group that has a C₁₋₃ main chain, and optionally has a C₁ branched chain. Specific examples of the C₁₋₃ alkyl group include methyl group, ethyl group, n-propyl group, i-propyl group, i-butyl group, and sec-butyl group, with methyl group, i-propyl group, i-butyl group, and sec-butyl group being preferred.

In formula (1), R³ is a -(CH₂)ₙ-X group. In the -(CH₂)ₙ-X group, n is a number from 1 to 4; and X is amino group, guanidino group, -CONH₂ group, or a 5-membered ring group or 6-membered ring group optionally having one to three nitrogen atoms or a fused heterocyclic group composed of the 5-membered ring and the 6-membered ring.

In the -(CH₂)ₙ-X group of R³, X is preferably amino group, guanidino group, carbamoyl group (-CONH₂ group), pyrrole group, imidazole group, pyrazole group, or indole group, and more preferably imidazole group. Furthermore, in the -(CH₂)ₙ-X group, n is preferably 1 or 2, and more preferably 1.

Thus, the -(CH₂)ₙ-X group is preferably aminomethyl group, 2-aminoethyl group, 3-aminopropyl group, 4-aminobutyl group, carbamoylmethyl group, 2-carbamoylethyl group, 3-carbamoylbutyl group, 2-guanidinoethyl group, 3-guanidinobutyl group, pyrrolemethyl group, 4-imidazolemethyl group, pyrazolemethyl group, or 3-indolemethyl group; more preferably 4-aminobutyl group, carbamoylmethyl group, 2-carbamoylethyl group, 3-guanidinobutyl group, 4-imidazolemethyl group, or 3-indolemethyl group; and still more preferably 4-imidazolemethyl group.

Particularly suitable examples of the lipid peptide compound of formula (1) include the following compounds each formed of a lipid moiety and a peptide moiety (amino acid assembly), wherein the amino acid abbreviations are alanine (Ala), asparagine (Asn), glutamine (Gln), glycine (Gly), histidine (His), isoleucine (Ile), leucine (Leu), lysine (Lys), tryptophan (Trp), and valine (Val): lauroyl-Gly-His, lauroyl-Gly-Gln, lauroyl-Gly-Asn, lauroyl-Gly-Trp, lauroyl-Gly-Lys, lauroyl-Ala-His, lauroyl-Ala-Gln, lauroyl-Ala-Asn, lauroyl-Ala-Trp, lauroyl-Ala-Lys; myristoyl-Gly-His, myristoyl-Gly-Gln, myristoyl-Gly-Asn, myristoyl-Gly-Trp, myristoyl-Gly-Lys, myristoyl-Ala-His, myristoyl-Ala-Gln, myristoyl-Ala-Asn, myristoyl-Ala-Trp, myristoyl-Ala-Lys; palmitoyl-Gly-His, palmitoyl-Gly-Gln, palmitoyl-Gly-Asn, palmitoyl-Gly-Trp, palmitoyl-Gly-Lys, palmitoyl-Ala-His, palmitoyl-Ala-Gln, palmitoyl-Ala-Asn, palmitoyl-Ala-Trp, palmitoyl-Ala-Lys; stearoyl-Gly-His, stearoyl-Gly-Gln, stearoyl-Gly-Asn, stearoyl-Gly-Trp, stearoyl-Gly-Lys, stearoyl-Ala-His, stearoyl-Ala-Gln, stearoyl-Ala-Asn, stearoyl-Ala-Trp, and stearoyl-Ala-Lys.

The most preferred examples of the lipid peptide compound of formula (1) include lauroyl-Gly-His, lauroyl-Ala-His-myristoyl-Gly-His, myristoyl-Ala-His; palmitoyl-Gly-His, palmitoyl-Ala-His; stearoyl-Gly-His, and stearoyl-Ala-His.

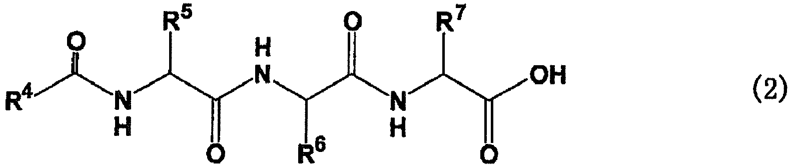

In formula (2), R⁴ is a C₉₋₂₃ aliphatic group, and preferred specific examples of R⁴ include the same groups as those defined by R¹ above.

In formula (2), R⁵ to R⁷ are each independently a hydrogen atom, a C₁₋₄ alkyl group optionally having a C₁ or C₂ branched chain, or a -(CH₂)ₙ-X group, and at least one of R⁵ to R⁷ is a -(CH₂)ₙ-X group. In formula (2), n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring. Preferred specific examples of R⁵ to R⁷ include the same groups as those defined by R² and R³ above.

Suitable examples of the lipid peptide compound of formula (2) include the following compounds each formed of a lipid moiety and a peptide moiety (amino acid assembly): myristoyl-Gly-Gly-His, myristoyl-Gly-Gly-Gln, myristoyl-Gly-Gly-Asn, myristoyl-Gly-Gly-Trp, myristoyl-Gly-Gly-Lys, myristoyl-Gly-Ala-His, myristoyl-Gly-Ala-Gln, myristoyl-Gly-Ala-Asn, myristoyl-Gly-Ala-Trp, myristoyl-Gly-Ala-Lys, myristoyl-Ala-Gly-His, myristoyl-Ala-Gly-Gln, myristoyl-Ala-Gly-Asn, myristoyl-Ala-Gly-Trp, myristoyl-Ala-Gly-Lys, myristoyl-Gly-His-Gly, myristoyl-His-GIy-Gly, palmitoyl-Gly-Gly-His, palmitoyl-Gly-Gly-Gln, palmitoyl-Gly-Gly-Asn, palmitoyl-Gly-Gly-Trp, palmitoyl-Gly-Gly-Lys, palmitoyl-Gly-Ala-His, palmitoyl-Gly-Ala-Gln, palmitoyl-Gly-Ala-Asn, palmitoyl-Gly-Ala-Trp, palmitoyl-Gly-Ala-Lys, palmitoyl-Ala-Gly-His, palmitoyl-Ala-Gly-Gln, palmitoyl-Ala-Gly-Asn, palmitoyl-Ala-Gly-Trp, palmitoyl-Ala-Gly-Lys, palmitoyl-Gly-His-Gly, and palmitoyl-His-Gly-Gly.

The most preferred examples of the above include lauroyl-Gly-Gly-His, myristoyl-Gly-Gly-His, palmitoyl-Gly-Gly-His, palmitoyl-Gly-His-Gly, palmitoyl-His-Gly-Gly, and stearoyl-Gly-Gly-His.

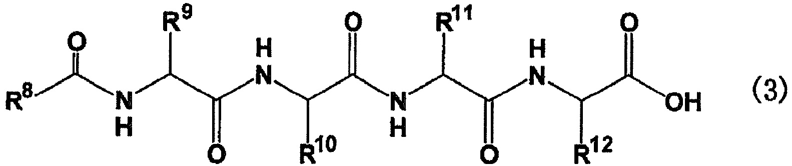

In formula (3), R⁸ is a C₉₋₂₃ aliphatic group, and preferred specific examples of R⁸ include the same groups as those defined by R¹ above.

In formula (3), R⁹ to R¹² are each independently a hydrogen atom, a C₁₋₄ alkyl group optionally having a C₁ or C₂ branched chain, or a -(CH₂)ₙ-X group, and at least one of R⁹ to R¹² is a -(CH₂)ₙ-X group. In formula (3), n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring. Preferred specific examples of R⁹ to R¹² include the same groups as those defined by R² and R³ above.

Thus, particularly suitable examples of the lipid peptide compound of formula (3) include lauroyl-Gly-Gly-Gly-His, myristoyl-Gly-Gly-Gly-His, palmitoyl-Gly-Gly-Gly-His, palmitoyl-Gly-Gly-His-Gly, palmitoyl-Gly-His-Gly-Gly, palmitoyl-His-Gly-Gly-Gly, and stearoyl-Gly-Gly-Gly-His.

In the present invention, the amount of the lipid peptide compound to be contained is, for example, 0.01 to 20% by mass, preferably 0.05 to 10% by mass, and more preferably 0.1 to 5% by mass, based on the total mass of the obtained solid base material for external use on skin.

In the present invention, the amount of the lipid peptide compound to be contained is, for example, 5 to 20% by mass, and preferably 10 to 20% by mass, based on the total mass of the obtained aqueous composition.

The lipid peptide compound to be used in the present invention comprises at least one of compounds (lipid peptides) of formulas (1) to (3) or pharmacologically usable salts thereof. These compounds may be used alone or in combination of two or more as a hydrogelator.

### [Surfactant]

As the surfactant to be used in the solid base material for external use on skin or the aqueous composition of the present invention, an ethylene glycol alkyl ether, a polyglycerin fatty acid ester, a polyoxyethylene polyoxypropylene alkyl ether or a phospholipid can be suitably used.

Examples include glycerophospholipids such as phosphatidylcholine, phosphatidylethanolamine, phosphatidylserine, phosphatidylinositol, phosphatidylglycerol, diphosphatidylglycerol (cardiolipin), and phosphatidic acid; lysoglycerophospholipids such as lysophosphatidylcholine (lysolecithin), lysophosphatidylethanolamine, lysophosphatidylserine, lysophosphatidylinositol, lysophosphatidylglycerol, and lysophosphatidic acid; sphingophospholipids such as sphingomyelin; and hydrogenated additives thereof. These phospholipids may be derived from animals or plants such as soybeans or egg yolks, or may be chemically or enzymatically synthesized.

Among the above-mentioned compounds, preferred examples include lysophosphatidylcholine (lysolecithin), lysophosphatidylethanolamine, lysophosphatidylserine, lysophosphatidylinositol, lysophosphatidylglycerol, and lysophosphatidic acid; and more preferred examples include lysophosphatidylcholine (lysolecithin).

Examples of the ethylene glycol alkyl ether include polyoxyethylene alkyl ethers, polyoxyethylene lauryl ethers, polyoxyethylene palmitoyl ethers, and polyoxyethylene stearyl ethers. Commercially available ethylene glycol alkyl ethers may also be used, and examples of such commercial products include those from the EMULGEN (registered trademark) series and the EMANON (registered trademark) series available from Kao Corporation; for example, EMULGEN 102KG, EMULGEN 103, EMULGEN 104P, EMULGEN 105, EMULGEN 106, EMULGEN 108, EMULGEN 109P, EMULGEN 120, EMULGEN 123P, EMULGEN 130K, EMULGEN 147, EMULGEN 150, EMULGEN 210P, EMULGEN 220, EMULGEN 306P, EMULGEN 320P, EMULGEN 350, EMULGEN 404, EMULGEN 408, EMULGEN 409PV, EMULGEN 420, EMULGEN 430, EMULGEN 705, EMULGEN 707, EMULGEN 709, EMULGEN 1108, EMULGEN 1118S-70, EMULGEN 1135S-70, EMULGEN 1150S-60, EMULGEN 4085, EMULGEN 2020G-HA, EMULGEN 2025G. EMANON 1112, EMANON 3199V, EMANON 3299V, EMANON 3299RV, and EMANON 4110. More preferred examples include EMULGEN 103, EMULGEN 104P, EMULGEN 105, EMULGEN 106, EMULGEN 108, EMULGEN 109P, EMULGEN 210P, EMULGEN 306P, EMULGEN 320P, EMULGEN 404, EMULGEN 408, EMULGEN 409PV, EMULGEN 420, EMULGEN 705, EMULGEN 707, EMULGEN 709, EMULGEN 1108, EMULGEN 2020G-HA, EMANON 1112, and EMANON 4110 from Kao Corporation. Still more preferred examples include EMULGEN 104P, EMULGEN 105, EMULGEN 106, EMULGEN 108, EMULGEN 210P, EMULGEN 306P, EMULGEN 408, EMULGEN 409PV, EMULGEN 705, EMULGEN 707, EMULGEN 709, EMULGEN 1108, EMULGEN 2020G-HA, EMANON 1112, and EMANON 4110 from Kao Corporation. Besides the above, a suitable commercial product may be selected from the NIKKOL (registered trademark) series available from Nikko Chemicals Co., Ltd. Examples of suitable products from the NIKKOL series include NIKKOL BT-5, NIKKOL BT-7, NIKKOL BT-9, and NIKKOL BT-12.

Examples of the polyglycerin fatty acid ester include glyceryl fatty acid partial esters such as glyceryl stearate, glyceryl isostearate, glyceryl palmitate, glyceryl myristate, glyceryl oleate, glyceryl cocoate, glycerin mono-cottonseed oil fatty acid, glycerin monoerucate, glycerin sesquioleate, glycerin α,α'-oleate pyroglutamate, and glycerin monostearate malate; polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate.

Examples of the polyoxyethylene polyoxypropylene alkyl ether include EMULGEN (registered trademark) LS-106, EMULGEN LS-110, EMULGEN LS-114, and EMULGEN MS-110 from Kao Corporation; and NIKKOL (registered trademark) PBC-31, NIKKOL PBC-33, NIKKOL PBC-34, NIKKOL PBC-41, NIKKOL PBC-44, NIKKOL PBN-4612, NIKKOL PBN-4620, and NIKKOL PBN-4630 from Nikko Chemicals Co., Ltd. Preferred examples include EMULGEN LS-106, EMULGEN LS-110, EMULGEN LS-114, and EMULGEN MS-110. More preferred examples include EMULGEN LS-106, EMULGEN LS-110, and EMULGEN MS-110.

As the surfactant to be used in the present invention, a surfactant having an HLB (Hydrophile-Lipophile Balance) value of 8 to 20 can be suitably used. A surfactant having an HLB value of 8 to 14 is more preferred.

Examples of such surfactants include sorbitan isostearate, steareth-8, beheneth-10, laureth-5, ceteth-7, oleth-8, PEG-8 glyceryl isostearate, choleth-10, PEG-10BG isostearate, PEG-30 glyceryl triisostearate, PEG-30 glyceryl triisostearate, PEG-30 glyceryl trioleate, PEG-30 trimethylolpropane triisostearate, PEG-30 hydrogenated castor oil laurate, PEG-30 hydrogenated castor oil PCA isostearate, octyldodeceth-10, PEG-12 dilaurate, sorbeth-40 tetraoleate, polyglyceryl-10 diisostearate, PEG-20 glyceryl diisostearate, PEG-8 isostearate, PEG-10 glyceryl isostearate, PEG-60 hydrogenated castor oil triisostearate, PPG-2-deceth-7, oleth-10, hydrogenated dimer dilinoleth-20, sorbitan cocoate, isosteareth-10, steareth-11, PEG-30 trimethylolpropane trimyristate, PEG-40 hydrogenated castor oil isostearate, PEG-40 hydrogenated castor oil isostearate, PEG-40 hydrogenated castor oil PCA isostearate, laureth-7, isoceteth-10, ceteth-10, PEG-10 isostearate, PEG-10 stearate, PEG-10 oleate, PEG-10 glyceryl stearate, oleth-12, decyltetradeceth-15, choleth-15, PEG-16 dilaurate, PEG-30 hydrogenated castor oil, PEG-40 glyceryl triisostearate, PEG-40 glyceryl trioleate, PEG-40 trimethylolpropane triisostearate, PEG-40 hydrogenated castor oil laurate, and PEG-12 laurate.

In the present invention, the amount of the surfactant to be contained is, for example, 0.01 to 20% by mass, preferably 0.05 to 10% by mass, and more preferably 0.1 to 5% by mass, based on the total mass of the obtained solid base material for external use on skin.

In the present invention, the amount of the surfactant to be contained is, for example, 1 to 20% by mass, and preferably 2 to 20% by mass, based on the total mass of the obtained aqueous composition.

The surfactant to be used in the present invention is at least one of the above-mentioned group of surfactants, and these surfactants may be used alone or in combination of two or more.

### [1,3-Alkanediol]

The 1,3-alkanediol to be used in the solid base material for external use on skin or the aqueous composition of the present invention has the function of enhancing the solubility of the lipid peptide compound.

Specific examples of the 1,3-alkanediol include 1,3-propanediol, 2-methyl-1,3-propanediol, 1,3-butanediol, 3-methyl-1,3-butanediol, 1,3-pentanediol, 1,3-hexanediol, 2-ethyl-1,3-hexanediol, 2-ethyl-1,3-octanediol, and 1,3-decanediol. Preferred examples include 1,3-pentanediol, 1,3-hexanediol, 2-ethyl-1,3-hexanediol, and 2-ethyl-1,3-octanediol. The 1,3-alkanediol to be used in the present invention is at least 2-ethyl-1,3-hexanediol, alone or in combination of two or more of the above-mentioned group of 1,3-alkanediols.

In the present invention, the amount of the 1,3-alkanediol to be contained is, for example, 1 to 20% by mass, preferably 0.01 to 10% by mass, and more preferably 0.1 to 5% by mass, based on the total mass of the obtained solid base material for external use on skin.

In the present invention, the amount of the 1,3-alkanediol to be contained is, for example, 1 to 20% by mass, and preferably 2 to 10% by mass, based on the total mass of the obtained aqueous composition.

### [Fatty Acid]

The solid base material for external use on skin or the aqueous composition of the present invention may further comprise a fatty acid. The fatty acid to be used in the present invention is preferably at least one selected from the group consisting of saturated and unsaturated fatty acids each having a carbon atom number of 10 to 20, as well as salts of these fatty acids. Examples of fatty acids include capric acid, undecanoic acid, lauric acid, tridecanoic acid, myristic acid, pentadecanoic acid, palmitic acid, margaric acid, and stearic acid. More preferred examples include capric acid, lauric acid, myristic acid, palmitic acid, and stearic acid; in particular, stearic acid is preferred.

In the present invention, the amount of the fatty acid to be contained is, for example, 0.01 to 2.0% by mass, and preferably 0.01 to 1.0% by mass, based on the total mass of the obtained solid base material for external use on skin.

In the present invention, the amount of the fatty acid to be contained is, for example, 0.5 to 5% by mass, and preferably 0.5 to 3% by mass, based on the total mass of the obtained aqueous composition.

The fatty acid to be used in the present invention is at least one of the above-mentioned group of fatty acids, and these fatty acids may be used alone or in combination of two or more.

### [Oleaginous Base]

The solid base material for external use on skin of the present invention may further comprise an oleaginous base. Likewise, the aqueous composition of the present invention may further comprise an oleaginous base. Preferred examples of the oleaginous base to be used in the present invention include higher (polyhydric) alcohols such as cetanol, myristyl alcohol, oleyl alcohol, lauryl alcohol, cetostearyl alcohol, stearyl alcohol, aralkyl alcohols, behenyl alcohol, jojoba alcohol, chimyl alcohol, selachyl alcohol, batyl alcohol, hexyldecanol, isostearyl alcohol, 2-octyldodecanol, and dimer diols; aralkyl alcohols such as benzyl alcohol and derivatives thereof; isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteiso-heneicosanoic acid, branched long-chain fatty acids, dimer acids, hydrogenated dimer acids, and the like; hydrocarbons such as liquid paraffin (mineral oil), heavy liquid isoparaffin, light liquid isoparaffin, α-olefin oligomers, polyisobutene, hydrogenated polyisobutene, polybutene, squalane, olive-derived squalane, squalene, vaseline, and solid paraffin; waxes such as candelilla wax, carnauba wax, rice wax, Japan wax, beeswax, montan wax, ozokerite, ceresin, paraffin wax, microcrystalline wax, petrolatum, Fischer-Tropsch Wax, polyethylene wax, and ethylene-propylene copolymers; vegetable oils and fats such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, and hydrogenated jojoba oil; animal oils and fats such as beef tallow, milk fat, horse fat, egg-yolk oil, mink oil, and turtle oil; animal waxes such as spermaceti, lanolin, and orange roughy oil; lanolins such as liquid lanolin, reduced lanolin, adsorption-purified lanolin, acetylated lanolin, acetylated liquid lanolin, hydroxylated lanolin, polyoxyethylene lanolin, lanolin fatty acids, hard lanolin fatty acids, lanolin alcohol, acetylated lanolin alcohol, and acetylated (cetyl/lanolyl) ester; sterols such as cholesterol, dihydrocholesterol, lanosterol, dihydrolanosterol, phytosterol, and cholic acid; sapogenins; saponins; sterol esters such as cholesteryl acetate, cholesteryl nonanoate, cholesteryl stearate, cholesteryl isostearate, cholesteryl oleate, di(cholesteryl/behenylloctyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acyl sarcosine alkyl esters such as isopropyl N-lauroylsarcosinate, cholesteryl 12-hydroxystearate, cholesteryl macadamiate, phytosteryl macadamiate, phytosteryl isostearate, soft lanolin fatty acid cholesteryl esters, hard lanolin fatty acid cholesteryl esters, branched long-chain fatty acid cholesteryl esters, and long-chain α-hydroxy fatty acid cholesteryl esters; lipid complexes such as phospholipid-cholesterol complexes and phospholipid-phytosterol complexes; monohydric alcohol esters of carboxylic acids such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters such as cetyl lactate, diisostearyl malate, and hydrogenated castor oil monoisostearate; polyhydric alcohol esters of fatty acids such as glyceryl trioctanoate (glyceryl tri-2-ethylhexanoate), glyceryl trioleate, glyceryl triisostearate, glyceryl diisostearate, caprylic/capric triglyceryl, caprylic/capric/myristic/stearic triglyceryl, hydrogenated rosin triglyceride (hydrogenated ester gum), rosin triglyceride (ester gum), glyceryl behenate eicosanedioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl ester, ditrimethylolpropane triethylhexanoate, ditrimethylolpropane isostearate/sebacate, pentaerythrityl triethylhexanoate, dipentaerythrityl hydroxystearate/stearate/rhodinate, diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, glycol distearate (ethylene glycol distearate), 3-methyl-1,5-pentanediol dineopentanoate, and 2,4-diethyl-1,5-pentanediol dineopentanoate; dimer acid derivatives or dimer diol derivatives such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensates, hydrogenated castor oil dimer dilinoleate, and hydroxyalkyl dimer dilinoleyl ether; fatty acid alkanolamides such as coconut oil fatty acid monoethanolamides (cocamide MEA), coconut oil fatty acid diethanolamides (cocamide DEA), lauric acid monoethanolamide (lauramide MEA), lauric acid diethanolamide (lauramide DEA), lauric acid monoisopropanolamide (lauramide MEPA), palmitic acid monoethanolamide (palmitamide MEA), palmitic acid diethanolamide (palmitamide DEA), and coconut oil fatty acid methylethanolamides (cocamide methyl MEA); silicones such as dimethicone (dimethylpolysiloxane), highly polymerized dimethicone (highly polymerized dimethylpolysiloxane), cyclomethicone (cyclic dimethylsiloxane, decamethylcyclopentasiloxane (also simply referred to as cyclopentasiloxane), phenyl trimethicone, diphenyl dimethicone, phenyl dimethicone, stearoxypropyldimethylamine, (aminoethylaminopropyl methicone/dimethicone) copolymers, dimethiconol, dimethiconol crosspolymers, silicone resin, silicone rubber, amino-modified silicones such as aminopropyl dimethicone and amodimethicone, cation-modified silicones, polyether-modified silicones such as dimethicone copolyols, polyglycerin-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymers; and fluorine oils such as perfluorodecane, perfluorooctane, and perfluoropolyether.

In the present invention, the amount of the oleaginous base to be contained is, for example, 1 to 50% by mass, preferably 5 to 50% by mass, and more preferably 10 to 50% by mass, based on the total mass of the obtained solid base material for external use on skin.

In the present invention, when the aqueous composition comprises an oleaginous base, the amount of the oleaginous base to be contained is, for example, 50 to 1% by mass, and preferably 30 to 1% by mass, based on the total mass of the aqueous composition.

The oleaginous base to be used in the present invention is at least one of the above-mentioned group of oleaginous bases, and these oleaginous bases may be used alone or in combination of two or more.

### [Organic Acid]

The solid base material for external use on skin of the present invention may further comprise an organic acid different from the fatty acid. Likewise, the aqueous composition of the present invention may further comprise an organic acid.

Examples of the organic acid include ascorbic acid, citric acid, lactic acid, glycolic acid, succinic acid, acetic acid, malic acid, tartaric acid, and fumaric acid. Among the above, preferred examples include ascorbic acid, citric acid, and lactic acid, and particularly preferred examples include ascorbic acid and citric acid.

In the present invention, the amount of the organic acid to be contained is, for example, 1 to 20% by mass, and preferably 1 to 10% by mass, based on the total mass of the obtained solid base material for external use on skin.

Furthermore, in the present invention, when the aqueous composition comprises an organic acid, the amount of the organic acid to be contained is, for example, 1 to 20% by mass, and preferably 1 to 10% by mass, based on the total mass of the aqueous composition.

### [Polyhydric Alcohol]

The solid base material for external use on skin or the aqueous composition of the present invention may comprise a polyhydric alcohol different from the 1,3-alkanediol. Examples of the polyhydric alcohol include glycerin, propylene glycol, and polyethylene glycol. The inclusion of the polyhydric alcohol can improve the temporal stability of the solid base material for external use on skin. Polyethylene glycol with an average molecular weight of 1,000 to 4,000, for example, can be suitably used as the polyethylene glycol.

In the present invention, the amount of the polyhydric alcohol to be contained is, for example, 1 to 70% by mass, and preferably 1 to 60% by mass, based on the total mass of the obtained solid base material for external use on skin.

Furthermore, in the present invention, when the aqueous composition comprises a polyhydric alcohol, the amount of the polyhydric alcohol to be contained is, for example, 1 to 40% by mass, and preferably 1 to 20% by mass, based on the total mass of the aqueous composition.

### [Polymeric Emulsifier]

The solid base material for external use on skin or the aqueous composition of the present invention may comprise a polymeric emulsifier. When a polymeric emulsifier is contained in the solid base material for external use on skin or the aqueous composition of the present invention, a gel can be formed even when the ingredients are stirred, i.e., cooled with stirring, during the preparation of the gel, and the physical performance of the gel can be improved, for example, the gel can be made more stable. Furthermore, heat-sensitive ingredients can be added to the solid base material for external use on skin or the aqueous composition of the present invention, which has been difficult when it is necessary to form a gel by allowing it to stand at high temperatures.

As the polymeric emulsifier, at least one polymer compound may be contained that is selected from the group consisting of graft polymer compounds each having a hydrophilic main chain and a hydrophobic moiety grafted thereto and block polymer compounds each composed of hydrophobic and hydrophilic structural units.

Examples of the graft polymer compounds each having a hydrophilic main chain and a hydrophobic moiety grafted thereto include xanthan gum, alginic acid esters, and cellulose derivatives.

Examples of the block polymer compounds each composed of hydrophobic and hydrophilic structural units include acrylic acid-alkyl methacrylate copolymers.

In particular, the polymeric emulsifier to be used in the solid base material for external use on skin is preferably a compound selected from the group consisting of carboxymethyl cellulose and alginic acid esters, and is particularly preferably propylene glycol alginate.

In the present invention, the amount of the polymeric emulsifier to be contained is, for example, 0.1 to 5% by mass, and preferably 0.1 to 1% by mass, based on the total mass of the obtained solid base material for external use on skin.

Furthermore, in the present invention, when the aqueous composition comprises a polymeric emulsifier, the amount of the polymeric emulsifier to be contained is, for example, 1 to 40% by mass, and preferably 1 to 20% by mass, based on the total mass of the aqueous composition.

### [Other Additives]

The solid base material for external use on skin or the aqueous composition of the present invention may contain additives generally usable as additives for cosmetics, quasi-drugs, and pharmaceuticals, as required. Examples of additive ingredients such as physiologically active substances and functional substances contained in external preparations for skin such as cosmetics, quasi-drugs, or pharmaceuticals include moisturizers, texture improvers, surfactants other than those mentioned above, polymers, thickeners/gelators, solvents, propellants, antioxidants, reducing agents, oxidizing agents, chelating agents, pH adjusters, acids, alkalis, powders, inorganic salts, ultraviolet absorbers, whitening agents, vitamins and derivatives thereof, hair growth-promoting agents, blood circulation promoters, stimulants, hormones, anti-wrinkle agents, anti-aging agents, firming agents, cooling agents, warming agents, wound-healing promoters, abirritants, analgesics, cell activators, plant/animal/microbial extracts, exfoliates/keratolytic agents, antiperspirants, refrigerants, astringents, enzymes, nucleic acids, perfumes, colors, coloring agents, dyes, pigments, antiphlogistics, anti-inflammatory agents, anti-asthmatic agents, anti-chronic obstructive pulmonary disease agents, anti-allergic agents, and immunomodulators.

The amount of these other additives to be contained may vary depending on the types of the additives; however, it is, for example, about 0.1 to 20% by mass or about 0.5 to 10% by mass, based on the total mass of the obtained solid base material for external use on skin.

Preferred examples of moisturizers and texture improvers include polyols and polymers thereof such as glycerin, trimethylolpropane, pentaerythritol, hexylene glycol, diglycerin, polyglycerin, diethylene glycol, dipropylene glycol, polypropylene glycol, and ethylene glycol-propylene glycol copolymers; glycol alkyl ethers such as diethylene glycol monoethyl ether (ethoxydiglycol), ethylene glycol monoethyl ether, ethylene glycol monobutyl ether, and diethylene glycol dibutyl ether; water-soluble esters such as polyglyceryl-10 (eicosanedioate/tetradecanedioate) and polyglyceryl-10 tetradecanedioate; sugar alcohols such as sorbitol, xylitol, erythritol, mannitol, and maltitol; saccharides and derivatives thereof such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, and γ-cyclodextrins, and modified cyclodextrins such as maltosyl cyclodextrin and hydroxyalkyl cyclodextrin), β-glucan, chitin, chitosan, heparin and derivatives thereof, pectin, arabinogalactan, dextrin, dextran, glycogen, ethyl glucoside, and glucosylethyl methacrylate polymer or copolymer; hyaluronic acid and sodium hyaluronate; sodium chondroitin sulfate; mucoitin sulfate, charonin sulfate, kerato sulfate, and dermatan sulfate; *Tremella fuciformis* extract and *Tremella fuciformis* polysaccharides; fucoidan; tuberose polysaccharides or natural polysaccharides; organic acids such as citric acid, tartaric acid, and lactic acid, as well as salts thereof; urea and derivatives thereof; 2-pyrrolidone-5-carboxylic acid and salts thereof such as sodium salt; amino acids such as betaine (trimethylglycine), proline, hydroxyproline, arginine, lysine, serine, glycine, alanine, phenylalanine, tyrosine, β-alanine, threonine, glutamic acid, glutamine, asparagine, aspartic acid, cystine, cysteine, methionine, leucine, isoleucine, valine, tryptophan, histidine, and taurine, as well as salts thereof; protein peptides and derivatives thereof such as collagen, fish collagen, atelocollagen, gelatin, elastin, peptides derived from degraded collagen, hydrolyzed collagen, hydroxypropylammonium chloride hydrolyzed collagen, peptides derived from degraded elastin, peptides derived from degraded keratin, hydrolyzed keratin, peptides derived from degraded conchiolin, hydrolyzed conchiolin, peptides derived from degraded silk protein, hydrolyzed silk, sodium lauroyl hydrolyzed silk, peptides derived from degraded soy protein, peptides derived from degraded wheat protein, hydrolyzed wheat protein, peptides derived from degraded casein, and acylated peptides; acylated peptides such as palmitoyl oligopeptide, palmitoyl pentapeptide, and palmitoyl tetrapeptide; silylated peptides; lactic acid bacteria culture, yeast extracts, eggshell membrane protein, bovine submaxillary mucin, hypotaurine, sesame lignan glycosides, glutathione, albumin, and whey; choline chloride and phosphorylcholine; animal and plant extract components such as placenta extract, elastin, collagen, aloe extract, *Hammamelis virginiana* water, *Luffa cylindrica* water, *Chamomilla recutita* extract, licorice extract, comfrey extract, silk extract, *Rosa roxburghii* extract, *Achillea millefolium* extract, *Eucalyptus globulus* extract, and melilot extract; and ceramides such as natural ceramides (types 1, 2, 3, 4, 5, and 6), hydroxyceramide, pseudoceramide, sphingoglycolipid, ceramide-containing extract, and glucosylceramide-containing extract.

Preferred examples of surfactants include anionic surfactants, nonionic surfactants, cationic surfactants, amphoteric surfactants, and polymer surfactants. Preferred examples of these surfactants are as follows: Preferred examples of anionic surfactants include fatty acid salts such as potassium laurate and potassium myristate; alkyl sulfates such as sodium lauryl sulfate, triethanolamine lauryl sulfate, and ammonium lauryl sulfate; polyoxyethylene alkyl sulfates such as sodium laureth sulfate and triethanolamine laureth sulfate; acyl N-methylamino acid salts such as sodium cocoyl methyl taurate, potassium cocoyl methyl taurate, sodium lauroyl methyl taurate, sodium myristoyl methyl taurate, sodium lauroyl methyl alaninate, sodium lauroyl sarcosinate, triethanolamine lauroyl sarcosinate, and sodium lauroyl glutamate methyl alaninate; acyl amino acid salts such as sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alaninate; polyoxyethylene alkyl ether acetates such as sodium laureth acetate; succinates such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates such as sodium hydrogenated coconut oil fatty acid glycerin sulfates; alkylbenzene polyoxyethylene sulfates; olefin sulfonates such as sodium α-olefin sulfonates; alkyl sulfosuccinates such as disodium lauryl sulfosuccinate and sodium dioctyl sulfosuccinate; alkyl ether sulfosuccinates such as disodium laureth sulfosuccinate, sodium monolauroyl monoethanolamide polyoxyethylene sulfosuccinate, and sodium lauryl polypropylene glycol sulfosuccinate; alkylbenzene sulfonates such as sodium tetradecylbenzene sulfonate and triethanolamine tetradecylbenzene sulfonate; alkyl naphthalene sulfonates; alkane sulfonates; α-sulfofatty acid methyl ester salts; acyl isethionates; alkyl glycidyl ether sulfonates; alkyl sulfoacetates; alkyl ether phosphates such as sodium laureth phosphate, sodium dilaureth phosphate, sodium trilaureth phosphate, and sodium monooreth phosphate; alkyl phosphates such as potassium lauryl phosphate; sodium caseinate; alkyl aryl ether phosphates; fatty acid amide ether phosphates; phospholipids such as phosphatidylglycerol, phosphatidylinositol, and phosphatidic acid; and silicone anionic surfactants such as carboxylic acid-modified silicones, phosphoric acid-modified silicones, and sulfuric acid-modified silicones. Preferred examples of nonionic surfactants include polyoxyethylene alkyl ethers with various numbers of polyoxyethylene units, such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyldodecyl ethers); polyoxyethylene alkyl phenyl ethers; castor oil derivatives and hydrogenated castor oil derivatives such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamate monoisostearate diester, and polyoxyethylene hydrogenated castor oil maleate; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers such as polyoxyethylene-polyoxypropylene cetyl ether, polyoxyethylene-polyoxypropylene 2-decyltetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols such as PPG-9 diglyceryl; glycerol fatty acid partial esters such as glyceryl stearate, glyceryl isostearate, glyceryl palmitate, glyceryl myristate, glyceryl oleate, glyceryl cocoate, glycerin mono-cottonseed oil fatty acid, glycerin monoerucate, glycerin sesquioleate, glycerin α,α'-oleate pyroglutamate, and glycerin monostearate malate; polyglycerin fatty acid esters such as polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono-fatty acid esters such as ethylene glycol monostearate; propylene glycol mono-fatty acid esters such as propylene glycol monostearate; pentaerythritol fatty acid partial esters; sorbitol fatty acid partial esters; maltitol fatty acid partial esters; maltitol ether; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; sugar derivative partial esters such as sucrose fatty acid esters, methyl glucoside fatty acid esters, and trehalose undecylenoate; alkyl glucosides such as caprylyl glucoside; alkyl polyglycosides; lanolin alcohol; reduced lanolin; polyoxyethylene fatty acid mono- and di-esters such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters, for example, polyoxyethylene monooleates such as polyoxyethylene glycerin monostearate, polyoxyethylene glycerin monoisostearate, and polyoxyethylene glycerin triisostearate; polyoxyethylene sorbitan fatty acid esters such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene-modified animal and vegetable fats and oils such as polyoxyethylene sorbitol beeswax; alkyl glyceryl ethers such as isostearyl glyceryl ether, chimyl alcohol, selachyl alcohol, and batyl alcohol; polyhydric alcohol alkyl ethers; polyoxyethylene alkylamines; tetrapolyoxyethylene/tetrapolyoxypropylene-ethylenediamine condensates; natural surfactants such as saponin and sophorolipid; polyoxyethylene fatty acid amides; fatty acid alkanolamides such as coconut oil fatty acid monoethanolamides (cocamide MEA), coconut oil fatty acid diethanolamides (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamides (cocamide methyl MEA); alkyl dimethylamine oxides such as lauramine oxide, cocamine oxide, stearamine oxide, and behenamine oxide; alkyl ethoxydimethylamine oxides; polyoxyethylene alkyl mercaptans; and silicone nonionic surfactants, for example, polyether-modified silicones such as dimethicone copolyols, polysiloxane-oxyalkylene copolymers, polyglycerin-modified silicones, and sugar-modified silicones. Preferred examples of cationic surfactants include alkyl trimethylammonium chlorides such as behentrimonium chloride, steartrimonium chloride, cetrimonium chloride, and lauryltrimonium chloride; alkyl trimethylammonium bromides such as steartrimonium bromide; dialkyl dimethylammonium chlorides such as distearyldimonium chloride and dicocodimonium chloride; fatty acid amide amines such as stearamidopropyl dimethylamine and stearamidoethyl diethylamine, as well as salts thereof; alkyl ether amines such as stearoxypropyldimethylamine and salts or quaternary salts thereof; fatty acid amide quaternary ammonium salts such as branched long-chain fatty acid (12 to 31) aminopropylethyldimethylammonium ethyl sulfates and lanolin fatty acid aminopropylethyldimethylammonium ethyl sulfates; polyoxyethylene alkylamines and salts or quaternary salts thereof; alkylamine salts; fatty acid amide guanidium salts; alkyl ether ammonium salts; alkyl trialkylene glycol ammonium salts; benzalkonium salts; benzethonium salts; pyridinium salts such as cetylpyridinium chloride; imidazolinium salts; alkyl isoquinolinium salts; dialkyl morpholinium salts; polyamine fatty acid derivatives; and silicone cationic surfactants such as amino-modified silicones such as aminopropyl dimethicone and amodimethicone, cation-modified silicones, cation-modified and polyether-modified silicones, and amino-modified and polyether-modified silicones. Preferred examples of amphoteric surfactants include N-alkyl-N,N-dimethylamino acid betaines such as lauryl betaine (lauryl dimethylaminoacetic acid betaine); fatty acid amido alkyl-N,N-dimethylamino acid betaines such as cocamidopropyl betaine and lauramidopropyl betaine; imidazoline betaines such as sodium cocoamphoacetate and sodium lauroamphoacetate; alkyl sulfobetaines such as alkyl dimethyltaurine; betaine sulfates such as alkyl dimethylaminoethanol sulfate; betaine phosphates such as alkyl dimethylaminoethanol phosphates; phospholipids such as phosphatidylcholine, phosphatidylethanolamine, phosphatidylserine, sphingophospholipids such as sphingomyelin, lysolecithin, hydrogenated soybean phospholipid, partially hydrogenated soybean phospholipid, hydrogenated egg yolk phospholipid, partially hydrogenated egg yolk phospholipid, and hydroxylated lecithin; and silicone amphoteric surfactants. Preferred examples of polymer surfactants include polyvinyl alcohol, sodium alginate, starch derivatives, tragacanth gum, and acrylic acid-alkyl methacrylate copolymers; and various silicone surfactants.

Polymers, thickeners, and gelators may be added in order to adjust the properties or feel upon use of the formulation, for example, to adjust the gel viscosity, achieve a moisturizing effect or a coating effect, or adjust the texture. Specific examples of polymers, thickeners, and gelators include guar gum, locust bean gum, quince seed, carrageenan, galactan, gum arabic, tara gum, tamarind, furcellaran, karaya gum, *Abelmoschus manihot,* cara gum, tragacanth gum, pectin, pectic acid and salts thereof such as sodium salt, alginic acid and salts thereof such as sodium salt, and mannan; starches such as rice starch, corn starch, potato starch, and wheat starch; xanthan gum, dextran, succinoglucan, curdlan, hyaluronic acid and salts thereof, xanthan gum, pullulan, gellan gum, chitin, chitosan, agar, brown algae extract, chondroitin sulfate, casein, collagen, gelatin, and albumin; celluloses and derivatives thereof such as methyl cellulose, ethyl cellulose, hydroxyethyl cellulose, hydroxypropyl cellulose, hydroxypropyl methylcellulose, carboxymethyl cellulose and salts thereof such as sodium salt, methylhydroxypropyl cellulose, sodium cellulose sulfate, dialkyldimethylammonium cellulose sulfate, crystalline cellulose, and cellulose powder; starch derivatives such as soluble starch, starch polymers such as carboxymethyl starch, methylhydroxypropyl starch, and methyl starch, starch hydroxypropyltrimonium chloride, and aluminum corn starch octenylsuccinate; alginic acid derivatives such as sodium alginate and propylene glycol alginate; polyvinylpyrrolidone (PVP), polyvinyl alcohol (PVA), vinylpyrrolidone-vinyl alcohol copolymers, and polyvinyl methyl ether; polyethylene glycol, polypropylene glycol, and polyoxyethylene-polyoxypropylene copolymers; amphoteric methacrylic acid ester copolymers such as (methacryloyloxyethylcarboxybetaine/alkyl methacrylate) copolymers and (acrylate/stearyl acrylate/ethylamine oxide methacrylate) copolymers; (dimethicone/vinyl dimethicone) crosspolymers, (alkyl acrylate/diacetone acrylamide) copolymers, and (alkyl acrylate/diacetone acrylamide) copolymers AMP; partially saponified polyvinyl acetate and maleic acid copolymers; vinylpyrrolidone-dialkylaminoalkyl methacrylate copolymers; acrylic resin alkanolamine; polyester and water-dispersible polyester; polyacrylamide; copolymers of polyacrylic esters such as polyethyl acrylate, carboxy vinyl polymers, polyacrylic acid and salts thereof such as sodium salt, and acrylic acid-methacrylic acid ester copolymers; acrylic acid-alkyl methacrylate copolymers; cationized celluloses such as polyquaternium-10, diallyldimethylammonium chloride-acrylamide copolymers such as polyquaternium-7, acrylic acid-diallyldimethylammonium chloride copolymers such as polyquaternium-22, acrylic acid-diallyldimethylammonium chloride-acrylamide copolymers such as polyquaternium-39, acrylic acid-cationized methacrylic acid ester copolymers, acrylic acid-cationized methacrylic acid amide copolymers, acrylic acid-methyl acrylate-methacrylamidopropyltrimethylammonium chloride copolymers such as polyquaternium-47, and methacryloyl chloride choline ester polymers; cationized polysaccharides such as cationized oligosaccharides, cationized dextran, and guar hydroxypropyltrimonium chloride; polyethyleneimine; cationic polymers; copolymers of 2-methacryloyloxyethyl phosphorylcholine and butyl methacrylate, such as polyquaternium-51; polymer emulsions such as acrylic resin emulsions, poly(ethyl acrylate) emulsions, polyacrylalkyl ester emulsions, polyvinyl acetate resin emulsions, natural rubber latex, and synthetic latex; nitrocellulose; polyurethanes and various copolymers thereof; various silicones; various silicone copolymers such as acrylic-silicone graft copolymers; various fluorine polymers; 12-hydroxystearic acid and salts thereof; dextrin fatty acid esters such as dextrin palmitate and dextrin myristate; and silicic anhydride, fumed silica (silicic anhydride ultrafine particles), magnesium aluminum silicate, magnesium sodium silicate, metallic soaps, metal dialkyl phosphates, bentonite, hectorite, organo-modified clay minerals, sucrose fatty acid esters, and fructooligosaccharide fatty acid esters. Among the above-mentioned examples, celluloses and derivatives thereof, alginic acid and salts thereof, polyvinyl alcohol, hyaluronic acid and salts thereof, and collagen are preferred.

Preferred examples of solvents and propellants include lower alcohols such as ethanol, 2-propanol (isopropyl alcohol), butanol, and isobutyl alcohol; glycols such as propylene glycol, diethylene glycol, dipropylene glycol, and isopentyldiol; glycol ethers such as diethylene glycol monoethyl ether (ethoxy diglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters such as ethylene glycol monoethyl ether acetate, diethylene glycol monoethyl ether acetate, and propylene glycol monoethyl ether acetate; glycol esters such as diethoxyethyl succinate and ethylene glycol disuccinate; benzyl alcohol, benzyloxyethanol, propylene carbonate, dialkyl carbonate, acetone, ethyl acetate, and N-methylpyrrolidone; toluene; fluorocarbons and next-generation fluorocarbons; and propellants such as LPG, dimethyl ether, and carbon dioxide gas.

Preferred examples of antioxidants include tocopherol (vitamin E) and tocopherol derivatives such as tocopherol acetate; BHT and BHA; gallic acid derivatives such as propyl gallate; vitamin C (ascorbic acid) and/or derivatives thereof; erythorbic acid and derivatives thereof; sulfites such as sodium sulfite; hydrogen sulfites such as sodium hydrogen sulfite; thiosulfates such as sodium thiosulfate; metabisulfites; thiotaurine and hypotaurine; thioglycerol, thiourea, thioglycolic acid, and cysteine hydrochloride.

Preferred examples of reducing agents include thioglycolic acid, cysteine, and cysteamine.

Preferred examples of oxidizing agents include hydrogen peroxide solution, ammonium persulfate, sodium bromate, and percarbonic acid.

Preferred examples of chelating agents include edetates (ethylenediamine tetraacetates) such as EDTA, EDTA-2Na, EDTA-3Na, and EDTA-4Na; hydroxyethylethylenediamine triacetates such as HEDTA-3Na; pentetates (diethylenetriamine pentaacetate); phytic acid; phosphonic acids such as etidronic acid and salts thereof such as sodium salt; polyamino acids such as polyaspartic acid and polyglutamic acid; sodium polyphosphate, sodium metaphosphate, and phosphoric acid; sodium citrate, citric acid, alanine, dihydroxyethylglycine, gluconic acid, ascorbic acid, succinic acid, and tartaric acid.

Preferred examples of pH adjusters, acids, and alkalis include citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-hydroxymethyl-1,3-propanediol, arginine, sodium hydroxide, potassium hydroxide, aqueous ammonia, guanidine carbonate, and ammonium carbonate.

Preferred examples of powders include inorganic powders having various sizes and shapes, such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstates, magnesium, zeolite, barium sulfate, calcined calcium sulfate, calcium phosphate, fluorapatite, hydroxyapatite, ceramic powder, bentonite, smectite, clay, mud, metallic soaps (for example, zinc myristate, calcium palmitate, and aluminum stearate), calcium carbonate, red iron oxide, yellow iron oxide, black iron oxide, ultramarine, prussian blue, carbon black, titanium oxide, titanium oxide fine particles and titanium oxide ultrafine particles, zinc oxide, zinc oxide fine particles and zinc oxide ultrafine particles, alumina, silica, fumed silica (silicic anhydride ultrafine particles), titanated mica, fish scale foil, boron nitride, photochromic pigments, synthetic fluorophlogopites, particulate composite powders, gold, and aluminum, as well as inorganic powders such as hydrophobic or hydrophilic powders obtained by treating the above-mentioned powders with various surface-treating agents such as silicones, for example, hydrogen silicone and cyclic hydrogen silicone, other silanes, or titanium coupling agents; and organic powders, surface-treated powders thereof, and organic-inorganic composite powders having various sizes and shapes, such as starches, celluloses, nylon powders, polyethylene powders, polymethyl methacrylate powders, polystyrene powders, styrene-acrylic acid copolymer resin powders, polyester powders, benzoguanamine resin powders, polyethylene terephthalate-polymethyl methacrylate laminated powders, polyethylene terephthalate-aluminum-epoxy laminated powders, urethane powders, silicone powders, and Teflon (registered trademark) powder.

Preferred examples of inorganic salts include sodium chloride-containing salts such as common salt, regular salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; and sodium phosphates such as mono-, di-, and tri-sodium phosphates, potassium phosphates, calcium phosphates, and magnesium phosphates.

Preferred examples of ultraviolet absorbers include benzoate ultraviolet absorbers such as p-aminobenzoic acid, p-aminobenzoic acid monoglycerin ester, N,N-dipropoxy-p-aminobenzoic acid ethyl ester, N,N-diethoxy-p-aminobenzoic acid ethyl ester, N,N-dimethyl-p-aminobenzoic acid ethyl ester, N,N-dimethyl-p-aminobenzoic acid butyl ester, and N,N-dimethyl-p-aminobenzoic acid ethyl ester; anthranilate ultraviolet absorbers such as homomenthyl-N-acetylanthranilate; salicylate ultraviolet absorbers such as salicylic acid and sodium salt thereof, amyl salicylate, methyl salicylate, homomenthyl salicylate, octyl salicylate, phenyl salicylate, benzyl salicylate, and p-isopropanolphenyl salicylate; cinnamate ultraviolet absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate (octyl p-methoxycinnamate), 2-ethoxyethyl-p-methoxycinnamate (cinoxate), cyclohexyl-p-methoxycinnamate, ethyl-α-cyano-β-phenylcinnamate, 2-ethylhexyl-α-cyano-β-phenyleinnamate (octocrylene), glyceryl mono-2-ethylhexanoyl-di-p-methoxycinnamate, ferulic acid, and derivatives thereof; benzophenone ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor and 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives such as 4-t-butyl methoxydibenzoylmethane; octyl triazone; urocanic acid and urocanic acid derivatives such as ethyl urocanate; and 2-(2'-hydroxy-5'-methylphenyl)benzotriazole, 1-(3,4-dimethoxyphenyl)-4,4-dimethyl-1,3-pentanedione, hydantoin derivatives such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of whitening agents include hydroquinone glycosides such as arbutin and α-arbutin, as well as esters thereof; ascorbic acid and ascorbic acid derivatives, for example, ascorbyl phosphates such as sodium ascorbyl phosphate and magnesium ascorbyl phosphate, ascorbyl fatty acid esters such as ascorbyl tetraisopalmitate, ascorbic acid alkyl ethers such as ascorbic acid ethyl ether, ascorbic acid glucosides such as ascorbic acid 2-glucoside and fatty acid esters thereof, ascorbyl sulfate, and tocopheryl ascorbyl phosphate; kojic acid, ellagic acid, tranexamic acid and derivatives thereof, ferulic acid and derivatives thereof, placenta extract, glutathione, oryzanol, butylresorecinol, and plant extracts such as oil-soluble *Chamomilla recutita* flower extract, oil-soluble licorice extract, *Tamarix chinensis* extract, and *Saxifraga stolonifera* extract.

Preferred examples of vitamins and derivatives thereof include forms of vitamin A such as retinol, retinol acetate, and retinol palmitate; forms of the vitamin B group such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acids, nicotinic acids such as nicotinamide and benzyl nicotinate, and cholines; forms of vitamin C such as ascorbic acid and salts thereof such as sodium salt; vitamin D; forms of vitamin E such as α-, β-, γ-, and δ-tocopherols; other vitamins such as pantothenic acid and biotin; ascorbic acid derivatives, for example, ascorbyl phosphates such as sodium ascorbyl phosphate and magnesium ascorbyl phosphate, ascorbyl fatty acid esters such as ascorbyl tetraisopalmitate, ascorbyl stearate, ascorbyl palmitate, and ascorbyl dipalmitate, ascorbic acid alkyl ethers such as ascorbic acid ethyl ether, ascorbic acid glucosides such as ascorbic acid 2-glucoside and fatty acid esters thereof, and tocopheryl ascorbyl phosphate; vitamin derivatives, for example, tocopherol derivatives such as tocopherol nicotinate, tocopherol acetate, tocopherol linoleate, tocopherol ferulate, and tocopherol phosphate, tocotrienol, and other various vitamin derivatives.

Preferred examples of hair growth-promoting agents, blood circulation promoters, and stimulants include plant extracts and tinctures such as swertia herb extract, capsicum tincture, ginger tincture, ginger extract, and cantharis tincture; capsaicin, nonylic acid vanillylamide, zingerone, ichthammol, tannic acid, bomeol, cyclandelate, cinnarizine, tolazoline, acetylcholine, verapamil, cepharanthine, γ-oryzanol, vitamin E and derivatives thereof such as tocopherol nicotinate and tocopherol acetate, γ-oryzanol, nicotinic acid and derivatives thereof such as nicotinamide, benzyl nicotinate, inositol hexanicotinate, and nicotinic alcohol, allantoin, Photosensitizer 301, Photosensitizer 401, carpronium chloride, pentadecanoic acid monoglyceride, flavanonol derivatives, stigmasterol or stigmastanol and glycosides thereof, and minoxidil.

Preferred examples of hormones include estradiol, estrone, ethinylestradiol, cortisone, hydrocortisone, and prednisone. Preferred examples of other medicinal agents such as anti-wrinkle agents, anti-aging agents, firming agents, cooling agents, warming agents, wound-healing promoters, abirritants, analgesics, and cell activators include retinols, retinoic acids, and tocopheryl retinoate; lactic acid, glycolic acid, gluconic acid, fruit acid, salicylic acid, and derivatives thereof such as glycosides and esters, and α- or β-hydroxy acids and derivatives thereof such as hydroxycapric acid, long-chain α-hydroxy fatty acids, and long-chain α-hydroxy fatty acid cholesteryl esters; γ-aminobutyric acid and γ-amino-β-hydroxybutyric acid; carnitine; carnocin; creatine; ceramides and sphingosines; caffeine, xanthine, and the like, as well as derivatives thereof; anti-oxidizing agents and active oxygen scavengers such as coenzyme Q10, carotene, lycopene, astaxanthin, lutein, α-lipoic acid, colloidal platinum nanoparticles, and fullerenes; catechins; flavones such as quercetin; isoflavones; gallic acid and sugar ester derivatives thereof; polyphenols such as tannin, sesamin, proanthocyanidin, chlorogenic acid, and apple polyphenols; rutin and derivatives thereof such as glycosides; hesperidin and derivatives thereof such as glycosides; lignan glycosides; licorice extract-related substances such as glabridin, glabrene, liquiritin, and isoliquiritin; lactoferrin; shogaol and gingerol; perfume substances such as menthol and cedrol as well as derivatives thereof; capsaicin, vanillin, and the like, as well as derivatives thereof; insect repellents such as diethyltoluamide; and complexes of physiologically active substances and cyclodextrins.

Preferred examples of plant, animal, and microbial extracts include iris extract, *Angelica keiskei* extract, *Thujopsis dolabrata* extract, asparagus extract, avocado extract, *Hydrangea serrata* leaf extract, almond extract, *Althea officinalis* root extract, *Arnica montana* extract, aloe extract, apricot extract, apricot kernel extract, ginkgo extract, *Artemisia capillaris* flower extract, fennel fruit extract, turmeric root extract, oolong tea extract, *uva-ursi* extract, rose fruit extract, *Echinacea angustifolia* leaf extract, *Isodonis japonicus* extract, *Scutellaria* root extract, *Phellodendron* bark extract, *Coptis* rhizome extract, barley extract, *Panax ginseng* extract, *Hypericum perforatum* extract, *Lamium album* extract, *Ononis spinosa* extract, *Nasturtium officinale* extract, orange extract, dried sea water residues, seaweed extract, Japanese persimmon leaf extract, *Pyracantha fortuneana* fruit extract, hydrolyzed elastin, hydrolyzed wheat powder, hydrolyzed silk, *Pueraria* root extract, *Chamomilla recutita* extract, oil-soluble *Chamomilla recutita* extract, carrot extract, *Artemisia capillaris* extract, *Avena fatua* extract, *Hibiscus sabdariffa* extract, licorice extract, oil-soluble licorice extract, kiwi fruit extract, kiou extract, *Auricularia auricula-judae* extract, *Cinchona* bark extract, cucumber extract, *Paulownia tomentosa* leaf extract, guanosine, guava extract, *Sophora* root extract, *Gardenia jasminoides* extract, *Sasa veitchii* extract, *Sophora flavescens* extract, walnut extract, chestnut extract, grapefruit extract, *Clematis vitalba* extract, black rice extract, black sugar extract, black vinegar, chlorella extract, mulberry extract, gentian extract, geranium herb extract, black tea extract, yeast extract, magnolia bark extract, coffee extract, burdock root extract, rice extract, fermented rice extract, fermented rice bran extract, rice germ oil, comfrey extract, collagen, bilberry extract, *Asiasarum* root extract, *Bupleurum* root extract, umbilical cord extract, saffron extract, salvia extract, *Saponaria officinalis* extract, bamboo grass extract, *Crataegus cuneata* fruit extract, *Bombyx mori excrementum* extract, *Zanthoxylum piperitum* peel extract, shiitake mushroom extract, *Rehmannia glutinosa* root extract, *Lithospermum* root extract, *Perilla frutescens* extract, *Tilia japonica* extract, *Filipendula multijuga* extract, jatoba extract, peony root extract, ginger extract, *Acorus calamus* root extract, *Betula alba* extract, *Tremella fuciformis* extract, *Equisetum arvense* extract, stevia extract, stevia fermentation product, *Tamarix chinensis* extract, *Hedera helix* extract, *Crataegus oxycantha* extract, *Sambucus nigra* extract, *Achillea millefolium* extract, *Mentha piperita* extract, sage extract, mallow extract, *Cnidium rhizome* extract, swertia herb extract, mulberry bark extract, rhubarb extract, soybean extract, jujubi extract, thyme extract, dandelion extract, lichens extract, tea extract, clove extract, *Imperata cylindrica* extract, *Citrus unshiu* peel extract, tea tree oil, *Rubus suavissimus* extract, capsicum extract, *Angelica acutiloba* root extract, *Calendula officinalis* extract, peach kernel extract, bitter orange peel extract, *Houttuynia cordata* extract, tomato extract, natto extract, carrot extract, garlic extract, *Rosa canina* fruit extract, hibiscus extract, *Ophiopogon* tuber extract, lotus extract, parsley extract, birch extract, honey, *Hamamelis virginiana* extract, *Parietaria officinalis* extract, *Rabdosia japonica* extract, bisabolol, cypress extract, *Bifidobacterium* extract, *Eriobotrya japonica* extract, *Tussilago farfara* extract, Japanese butterbur flower-bud extract, *Poria cocos sclerotium* extract, butcher's broom extract, grape extract, grape seed extract, propolis, *Luffa cylindrica* extract, safflower extract, peppermint extract, *Tilia miqueliaria* extract, *Paeonia suffruticosa* extract, hop extract, *Rosa rugosa* extract, pine extract, *Aesculus hippocastanum* extract, *Lysichiton camtschatcense* extract, *Sapindus mukurossi* extract, *Melissa officinalis* extract, *Nemacystus decipiens* extract, peach extract, cornflower extract, *Eucalyptus globulus* extract, *Saxifraga stolonifera* extract, *Citrus junos* extract, lily extract, coix seed extract, *Artemisia princeps* extract, lavender extract, green tea extract, egg shell membrane extract, apple extract, rooibos tea extract, *Litchi chinensis* extract, lettuce extract, lemon extract, *Forsythia* fruit extract, *Astragalus sinicus* extract, rose extract, rosemary extract, *Anthemis nobilis* extract, royal jelly extract, and *Sanguisorba officinalis* extract.

Examples of exfoliates/keratolytic agents include salicylic acid, sulfur, resorcin, selenium sulfide, and pyridoxine.

Examples of antiperspirants include aluminum chlorohydrate, aluminum chloride, zinc oxide, and zinc p-phenolsulfonate.

Examples of refrigerants include menthol and methyl salicylate.

Examples of astringents include citric acid, tartaric acid, lactic acid, aluminum potassium sulfate, and tannic acid.

Examples of enzymes include superoxide dismutase, catalase, lysozyme chloride, lipase, papain, pancreatin, and protease.

Preferred examples of nucleic acids include ribonucleic acids and salts thereof, deoxyribonucleic acids and salts thereof, and adenosine triphosphate disodium.

Preferred examples of perfumes include synthetic and natural perfumes as well as various perfume blends, such as acetyl cedrene, amylcinnamaldehyde, allylamyl glycolate, β-ionone, Iso E Super, isobutylquinoline, iris oil, irone, indole, ylang-ylang oil, undecanal, undecenal, γ-undecalactone, estragole, eugenol, oakmoss, opoponax resinoid, orange oil, eugenol, aurantiol, galaxolide, carvacrol, L-carvone, camphor, canon, carrot seed oil, clove oil, methyl cinnamate, geraniol, geranyl nitrile, isobornyl acetate, geranyl acetate, dimethylbenzylcarbinyl acetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinen, triplal, nerol, nonanal, 2,6-nonadienal, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexylcinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methylnonylacetaldehyde, γ-methylionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil, and various essential oils.

Preferred examples of colors, coloring agents, dyes, and pigments include certified colors such as Brown No. 201, Black No. 401, Violet No. 201, Violet No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green. No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407, and Yellow No. 5; other acid dyes such as Acid Red 14; basic dyes such as Arianor Sienna Brown, Arianor Madder Red, Arianor Steel Blue, and Arianor Straw Yellow; nitro dyes such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2, and Basic Blue 26; disperse dyes; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide and low-order titanium oxide; inorganic violet pigments such as mango violet and cobalt violet; inorganic green pigments such as chromium oxide, chromium hydroxide, and cobalt titanate; inorganic blue pigments such as ultramarine and prussian blue; pearl pigments such as titanium oxide-coated mica, titanium oxide-coated bismuth oxychloride, titanium oxide-coated talc, colored titanium oxide-coated mica, bismuth oxychloride, and fish scale foil; metal powder pigments such as aluminum powder, copper powder, and gold; surface-treated inorganic and metal powder pigments; organic pigments such as zirconium lake, barium lake, and aluminum lake; surface-treated organic pigments; natural colors and dyes, for example, anthraquinones such as astaxanthin and alizarin, naphthoquinones such as anthocyanidin, β-carotene, catenal, capsanthin, chalcone, carthamin, quercetin, crocin, chlorophyll, curcumin, cochineal, and shikonin, bixin, flavones, betacyanidine, henna, hemoglobin, lycopene, riboflavin, and rutin; oxidation dye intermediates and couplers such as p-phenylenediamine, toluene-2,5-diamine, o-, m-, and p-aminophenols, m-phenylenediamine, 5-amino-2-methylphenol, resorcin, 1-naphthol, and 2,6-diaminopyridine, as well as salts thereof; autoxidation dyes such as indoline; and dihydroxyacetone.

Preferred examples of antiphlogistics and anti-inflammatory agents include glycyrrhizic acid and derivatives thereof, glycyrrhetic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphenhydramine hydrochloride, and chlorpheniramine maleate; and plant extracts such as peach leaf extract and *Artemisia princeps* leaf extract.

Preferred examples of anti-asthmatic agents, anti-chronic obstructive pulmonary disease agents, anti-allergic agents, and immunomodulators include aminophylline, theophyllines, steroids (such as fluticasone and beclomethasone), leukotriene antagonists, thromboxane inhibitors, Intal, β2 agonists (such as formoterol, salmeterol, albuterol, tulobuterol, clenbuterol, and epinephrine), tiotropium, ipratropium, dextromethorphan, dimemorfan, bromhexine, tranilast, ketotifen, azelastine, cetirizine, chlorpheniramine, mequitazine, tacrolimus, ciclosporin, sirolimus, methotrexate, cytokine modulators, interferon, omalizumab, and protein/antibody preparations.

In addition to these ingredients, the solid base material for external use on skin and the aqueous composition of the present invention may contain known cosmetic ingredients, known pharmaceutical ingredients, and known food ingredients, such as ingredients described in Japanese Standards of Cosmetic Ingredients, Japanese Cosmetic Ingredients Codex, List of Cosmetics Ingredients Japanese Labelling Names issued by the Japan Cosmetic Industry Association, INCI dictionary (The International Cosmetic Ingredient Dictionary and Handbook), Japanese Standards of Quasi-drug Ingredients, Japanese Pharmacopoeia, Japanese Pharmaceutical Excipients, Japan's Specifications and Standards for Food Additives, and other standards, as well as ingredients described in Japanese and foreign patent publications and patent application publications (including Japanese translations of PCT international application publications and re-publications of PCT international publications) classified as International Patent Classification IPC classes A61K7 and A61K8, in known combinations and in known ratios and amounts.

### [Method for Producing Solid Base Material for External Use on Skin (1)]

The solid base material for external use on skin of the present invention can be produced using a lipid peptide compound comprising at least one of compounds of formulas (1) to (3) or pharmacologically usable salts thereof, a a surfactant being one or more compounds selected from ethylene glycol alkyl ethers, phospholipids, polyglycerin fatty acid esters and polyoxyethylene polyoxypropylene alkyl ethers, water, a 2-ethyl-1,3-hexanediol, as well as optionally a fatty acid, an oleaginous base, an organic acid, a polyhydric alcohol, a polymeric emulsifier, and other additives, which are mixed with stirring while heating, and then allowed to cool. As described below, the aqueous composition of the present invention can be produced during this production process.

For example, the solid base material for external use on skin of the present invention is produced by the following steps:
a) mixing the lipid peptide compound, a a surfactant being one or more compounds selected from ethylene glycol alkyl ethers, phospholipids, polyglycerin fatty acid esters and polyoxyethylene polyoxypropylene alkyl ethers, water, a 2-ethyl-1,3-hexanediol, and heating the mixture to prepare a solution or dispersion;
b) adding the solution or dispersion to water, and heating the mixture to a temperature not lower than room temperature and lower than 100°C; and
c) cooling the mixture, with stirring, to a temperature lower than the temperature in the above-described heating step, and then allowing the mixture to cool, thereby forming a gel solid (solid base material for external use on skin).

The fatty acid, the oleaginous base, the organic acid, the polyhydric alcohol, the polymeric emulsifier, and other additives may be added in the step of preparing the solution or dispersion in step a), or may be added in advance to the water to which the solution or dispersion is to be added in step b).

The amount of water is preferably 25% by mass or more and less than 99% by mass, based on the total mass of the obtained solid base material for external use on skin.

Furthermore, the amount of water is preferably 20% by mass or more and less than 80% by mass, based on the total mass of the obtained solution or dispersion.

In steps a) and b), the heating temperature is preferably 50 to 90°C, and more preferably 60 to 90°C, for example, 80°C. Stirring is preferably performed while heating. The heating and stirring time in each of these steps varies depending on the types and amounts of the lipid peptide compound and other ingredients such as a surfactant used; typically, these ingredients can be dissolved or dispersed in about 5 to 50 minutes.

Subsequent to steps a) and b), the mixture is cooled with stirring until the liquid temperature of the mixture becomes lower than the temperature in step b) (step c)). The cooling temperature is, for example, from room temperature to about 80°C, from room temperature to about 60°C, or from room temperature to about 40°C.

### [Methods for Producing Aqueous Composition and Solid Base Material for External Use on Skin (2)]

The method for producing the solid base material for external use on skin using the aqueous composition of the present invention will be hereinafter described.

As described in detail below, the aqueous composition is produced by step a) in [Method for Producing Solid Base Material for External Use on Skin (1)] above.

### <Method for Producing Aqueous Composition>

To produce the aqueous composition, initially, a lipid peptide compound comprising at least one of compounds of formulas (1) to (3) or pharmacologically usable salts thereof, a surfactant being one or more compounds selected from ethylene glycol alkyl ethers, phospholipids, polyglycerin fatty acid esters and polyoxyethylene polyoxypropylene alkyl ethers, water, and a 2-ethyl-1,3-hexanediol are mixed, and the mixture is heated to prepare a solution or dispersion. During the preparation of the solution or dispersion, a fatty acid and a polyhydric alcohol may be optionally added, and an oleaginous base, an organic acid, a polymeric emulsifier, and other additives may also be added.

Then, this solution or dispersion is cooled. As a result, the aqueous composition can be obtained.

The heating temperature is preferably 50 to 90°C, and more preferably 60 to 90°C, for example, 80°C. Stirring is preferably performed during heating. The heating (stirring) time varies depending on the types and amounts of the lipid peptide compound and other ingredients such as a surfactant used; typically, the heating (stirring) time is from about 5 to 50 minutes. As a result, a solution or dispersion in which the mixed ingredients are dissolved or dispersed is obtained.

It is preferred to cool, with stirring, the obtained solution or dispersion to a temperature lower than the heating temperature, for example, from room temperature to about 80°C, from room temperature to about 60°C, or from room temperature to about 40°C, and then stop the stirring and allow the solution or dispersion to stand.

The amount of water is preferably 20% by mass or more and less than 80% by mass, based on the total mass of the obtained aqueous composition.

The aqueous composition thus obtained is useful as a premix for preparing the solid base material for external use on skin. As described below, because the composition contains water and other ingredients such as an active ingredient, the solid base material for external use on skin can be readily prepared.

### [Method for Producing Solid Base Material for External Use on Skin Using Aqueous Composition]

The solid base material for external use on skin can be prepared using the thus-obtained aqueous composition of the present invention, for example, through the following steps 1) to 3):
1) a heating step of heating the aqueous composition to a temperature not lower than room temperature and lower than 100°C;
2) a mixing step of adding the heated aqueous composition to an aqueous phase heated to a temperature not lower than room temperature and lower than 100°C, followed by mixing, or adding an aqueous phase heated to a temperature not lower than room temperature and lower than 100°C to the heated aqueous composition, followed by mixing; and
3) a cooling step of cooling the resulting mixture to form a gel.

The aqueous phase contains water, may further contain an oleaginous base, a polyhydric alcohol, and a polymeric emulsifier, and may additionally contain a fatty acid, an organic acid, and other additives.

When the solid base material for external use on skin contains an organic acid, the solid base material for external use on skin containing the organic acid can be produced, for example, through the following steps 4) to 6):
4) a heating step of heating the aqueous composition to a temperature not lower than room temperature and lower than 100°C;
5) a mixing step of adding the heated aqueous composition to an aqueous phase heated to a temperature not lower than room temperature and lower than 100°C, followed by mixing, or adding an aqueous phase heated to a temperature not lower than room temperature and lower than 100°C to the heated aqueous composition, followed by mixing; and
6) a cooling step of cooling the resulting mixture to form a gel, wherein a mixed solution of water and an organic acid is added to the mixture during the cooling.

The aqueous phase contains water, may further contain an oleaginous base, a polyhydric alcohol, and a polymeric emulsifier, and may additionally contain a fatty acid and other additives.

The heating temperature for the aqueous composition in steps 1) and 4) is preferably 50 to 90°C, and more preferably 60 to 90°C, for example, 70 or 80°C. These steps are preferably performed with stirring. The heating (stirring) time in each of these steps varies depending on the types and amounts of the lipid peptide compound and other ingredients such as a surfactant contained in the aqueous composition; typically, the heating (stirring) time is from about 5 to 50 minutes. Through these steps, the aqueous composition is homogeneously dissolved.

The heating temperature for the aqueous phase in steps 2) and 5) is preferably 50 to 90°C, and more preferably 60 to 90°C, for example, 70 or 80°C. In particular, when the aqueous phase contains other ingredients such as an oleaginous base, the aqueous phase is preferably heated with stirring. Furthermore, when the aqueous phase contains an oleaginous base, a polyhydric alcohol, and a polymeric emulsifier, as well as an organic acid, a fatty acid, and other additives, the aqueous phase is preferably heated (with stirring) typically for about 5 to 50 minutes, until these ingredients are homogeneously dissolved or dispersed. The heating temperature for the aqueous phase may be the same as the heating temperature for the aqueous composition.

Subsequently in steps 3) and 6), the mixture obtained in the preceding step is cooled to form a gel. In this case, the mixture may be cooled with stirring. When the mixture is cooled with stirring, it is preferred to perform the stirring until the cooling temperature reaches, for example, room temperature to 80°C or room temperature to 60°C, for example, about 60°C, and then stop the stirring and allow the mixture to cool. It is particularly preferred to stop the stirring at 50°C or lower, and allow the mixture to cool.

Furthermore, when an organic acid such as ascorbic acid is to be contained in the solid base material for external use on skin, the method for producing the solid base material for external use on skin further includes the step of adding a mixed solution of water and an organic acid to the mixture during step 6) (cooling process), followed by mixing.

In this step, the mixed solution of water and an organic acid to be added preferably has substantially the same temperature as that of the mixture to which the mixed solution is to be added, so as to achieve homogeneous mixing. Furthermore, the mixed solution may also optionally contain an oleaginous base, a fatty acid, a polyhydric alcohol, a polymeric emulsifier, and other additives. In this case, the mixed solution may be heated (with stirring) at a suitable temperature until these ingredients are homogeneously dissolved or dispersed.

For example, when the liquid temperature of the mixture has become about 60°C while the mixture is being stirred in step 6), the mixed solution of water and an organic acid having a liquid temperature of about 60°C is added to the mixture, and then the mixture is mixed to give a homogeneous system. Then, it is preferred to stop the stirring, and allow the mixture to cool. As a result, a gel (solid base material for external use on skin) can be obtained.

Likewise, in the solid base material for external use on skin thus obtained using the aqueous composition, the amount of water to be contained therein is preferably 25% by mass or more and less than 99% by mass, based on the total mass of the solid base material for external use on skin.

### Examples

The present invention will be hereinafter described in detail with reference to examples and test examples.

### [Synthesis Example 1: Synthesis of Lipid Peptide (N-Palmitoyl-Gly-His)]

The lipid peptide used as a gelator in the examples was synthesized in accordance with the following method.

A 500-mL four-necked flask was charged with 14.2 g (91.6 mmol) of histidine, 30.0 g (91.6 mmol) of N-palmitoyl-Gly-methyl, and 300 g of toluene, and then 35.3 g (183.2 mmol) of a 28% methanol solution of sodium methoxide as a base was added thereto. The mixture was heated to 60°C in an oil bath, and continuously stirred for 1 hour. The oil bath was then removed, and the solution was allowed to cool to 25°C. To the solution, 600 g of acetone was added to cause reprecipitation, and the precipitate was collected by filtration. The obtained solid was dissolved in a mixed solution of 600 g of water and 750 g of methanol. To the solution, 30.5 ml (183.2 mmol) of 6N hydrochloric acid was added to neutralize the solution and precipitate a solid, and the solid was collected by filtration. Next, the obtained solid was dissolved in a mixed solution of 120 g of tetrahydrofuran and 30 g of water at 60°C, and then 150 g of ethyl acetate was added thereto. The mixture was cooled from 60 to 30°C. The precipitated solid was then collected by filtration. Furthermore, the obtained solid was dissolved in a solvent of 120 g of tetrahydrofuran and 60 g of acetonitrile. The solution was heated to 60°C, stirred for 1 hour, and then cooled. The solid was then collected by filtration. The obtained solid was washed with 120 g of water, collected by filtration, and then dried under reduced pressure to give 26.9 g of the free form of N-palmitoyl-Gly-His (hereinafter also simply referred to as Pal-GH) as white crystals (yield 65%).

### [Examples 1 to 3, and Comparative Examples 1 and 2]

### (Method for Preparing Solid Base Materials for External Use on Skin)

The ingredients of Phase A were weighed out in the proportions shown in Table 1 below (total amount: 25 g). The weighed-out Phase A was heated to 80°C to homogeneously dissolve all the ingredients.

The ingredients of Phase B shown in Table 1 below were weighed out and heated to 80°C. The previously prepared Phase A was gradually added to Phase B, and the mixture was stirred with heating to give a homogeneous solution.

The resulting solution was allowed to cool at room temperature.

### <Method for Measuring Breaking Strength>

The solid base materials for external use on skin were measured for breaking strength with YAMADEN RHEONER II CREEP METER RE2-33005B (from Yamaden Co., Ltd.), using a measurement rate of 0.5 mm/sec, a measurement distortion factor of 20%, a storing pitch of 0.10 sec, and the jig 30349-3.

The obtained results are shown in Table 1.

**Table 1**

| Components (g) | | | Example 1 | Example 2 | Example 3 |
|---|---|---|---|---|---|
| Phase A | Pal-GH | 20 wt% | 5.0 | 5.0 | 5.0 |
| | Polyoxyethylene Lauryl Ether^{*1} | 16 wt% | 4.0 | 4.0 | 4.0 |
| | 2-Ethyl-1,3-Hexanediol^{*2} | 8 wt% | 2.0 | 2.0 | 2.0 |
| | Propylene Glycol^{*3} | 20 wt% | 5.0 | 5.0 | 5.0 |
| | Stearic Acid^{*4} | 1 wt% | 0.25 | 0.25 | 0.25 |
| | Purified Water | 35 wt% | 8.75 | 8.75 | 8.75 |
| | Phase A, Total | wt% | 25 | 25 | 25 |
| Phase B | Purified Water | | 75 | 25 | 25 |
| | Propylene Glycol | | | 50 | |
| | Mineral Oil^{*5} | | | | 50 |
| All Ingredients, Total | | | 100 | 100 | 100 |
| Breaking Strength [×10⁵ Pa] | | | 1.60 | 1.50 | 1.20 |

| | | | | | |
|---|---|---|---|---|---|
| *1: from Nikko Chemicals Co., Ltd. *2: from KH Neochem Co., Ltd. *3: from Junsei Chemical Co., Ltd. *4: from Kao Corporation [trade name: Lunac S-98] *5: from PINOA Co., Ltd. | | | | | |

### [Examples 4 to 6]

### (Method for Preparing Solid Base Materials for External Use on Skin)

The ingredients of Phase A were weighed out in accordance with Table 2 below. The weighed-out Phase A was heated to 80°C to homogeneously dissolve all the ingredients.

The ingredients of Phase B shown in Table 2 below were weighed out and heated to 80°C. The previously prepared Phase A was gradually added to Phase B, and the mixture was stirred with heating to give a homogeneous solution. Then, the solution was cooled at room temperature, with stirring, to 50°C.

Into a screw tube No. 7 (from Maruemu Corporation), 40 g of the obtained solution was transferred, and allowed to cool at room temperature.

Each of the obtained base materials was visually evaluated using a test tube inversion method. Specifically, when the screw tube was inverted and the base material did not drop because it had lost the flowability of the solution, then the base material was evaluated as a "gel (O)". When the base material was dropped by gravity, and thus, did not form a gel, then the base material was evaluated as "×". The results are shown in Table 2.

**Table 2**

| Components (g) | | | Example 4 | Example 5 | Example 6 | |
|---|---|---|---|---|---|---|
| Phase A | Pal-GH | 20 wt% | 2.0 | 1.0 | 0.60 | |
| | Polyoxyethylene Lauryl Ether^{*1} | 16 wt% | 1.6 | 0.8 | 0.48 | |
| | 2-Ethl-1,3-Hexanediol^{*2} | 8 wt% | 0.8 | 0.4 | 0.24 | |
| | Propylene Glycol^{*3} | 20 wt% | 2.0 | 1.0 | 0.60 | |
| | Stearic Acid^{*4} | 1 wt% | 0.1 | 0.05 | 0.03 | |
| | Purified Water | 35 wt% | 3.5 | 1.75 | 1.05 | |
| | Phase A, Total | 100 wt% | 10 | 5 | 3 | |
| Phase B | Propylene Glycol Alginate^{*6} | | 0.3 | 0.3 | 0.3 | |
| | Purified Water | | 89.7 | 94.7 | 96.7 | |
| All Ingredients, Total | | | 100 | 100 | 100 | |
| Gelation Ability | | | ○ | ○ | ○ | |

| | | | | | | |
|---|---|---|---|---|---|---|
| *1: from Nikko Chemicals Co., Ltd. *2: from KH Neochem Co., Ltd. *3: from Junsei Chemical Co., Ltd. *4: from Kao Corporation [trade name: Lunac S-98] *6: from KIMICA Corporation [trade name: KIMILOID HV] | | | | | | |

As shown in Table 1, the solid base materials for external use on skin according to Examples 1 to 3 had breaking strengths of 1.2 to 1.6 × 10⁵ Pa; in particular, these solid base materials had breaking strengths required as stick-shaped base materials, i.e., had strengths sufficiently high to achieve adequate applicability, and showed substantially no variations in hardness depending on the solvent (Phase B).

Furthermore, as shown in Table 2, when Phases A as aqueous compositions were appropriately diluted, very soft gel base materials that had lost flowability were formed.

## Claims

1. A solid base material for external use on skin comprising:
a surfactant being one or more compounds selected from the group consisting of ethylene glycol alkyl ethers, phospholipids, polyglycerin fatty acid esters, and polyoxyethylene polyoxypropylene alkyl ethers;
water;
a 2-ethyl-1,3-hexanediol; and
a lipid peptide compound comprising at least one of compounds of formulas (1) to (3): 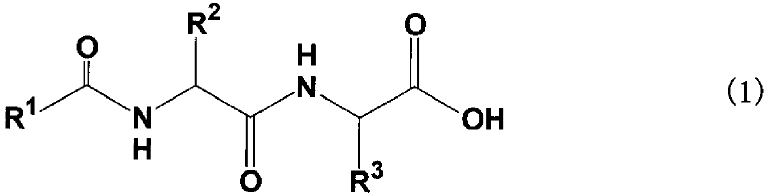
(wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); 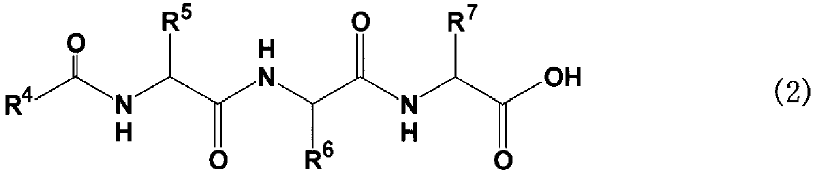 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); 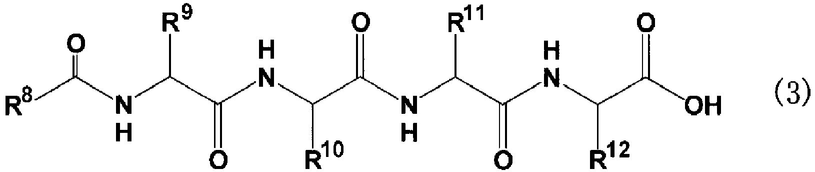 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); or
pharmacologically usable salts thereof.

2. The solid base material for external use on skin according to claim 1, which further comprises at least one fatty acid.

3. The solid base material for external use on skin according to claim 1 or 2, which further comprises at least one oleaginous base.

4. The solid base material for external use on skin according to any one of claims 1 to 3, which further comprises at least one organic acid different from the fatty acid.

5. The solid base material for external use on skin according to any one of claims 1 to 4, which further comprises at least one polyhydric alcohol different from the 2-ethyl-1,3-hexanediol.

6. The solid base material for external use on skin according to any one of claims 1 to 5, which further comprises at least one polymeric emulsifier.

7. The solid base material for external use on skin according to any one of claims 2 to 6, wherein the fatty acid is stearic acid.

8. The solid base material for external use on skin according to any one of claims 5 to 7, wherein the polyhydric alcohol is glycerin, propylene glycol, or polyethylene glycol.

9. The solid base material for external use on skin according to any one of claims 6 to 8, wherein the polymeric emulsifier is at least one polymer compound selected from the group consisting of graft polymer compounds each having a hydrophilic main chain and a hydrophobic moiety grafted thereto and block polymer compounds each composed of hydrophobic and hydrophilic structural units.

10. The solid base material for external use on skin according to any one of claims 1 to 9, which is used for cosmetics or pharmaceuticals.

11. The solid base material for external use on skin according to any one of claims 1 to 10, which is stick-shaped.

12. An aqueous composition comprising:
a surfactant being one or more compounds selected from the group consisting of ethylene glycol alkyl ethers, phospholipids, polyglycerin fatty acid esters, and polyoxyethylene polyoxypropylene alkyl ethers;
water;
a 2-ethyl-1,3-hexanediol; and
a lipid peptide compound comprising at least one of compounds of formulas (1) to (3): 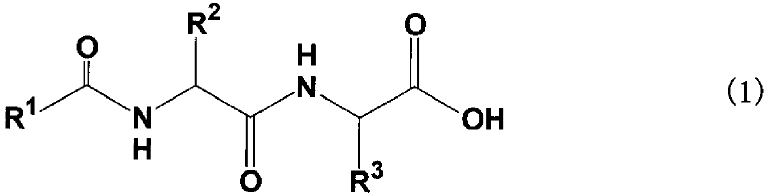
(wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); 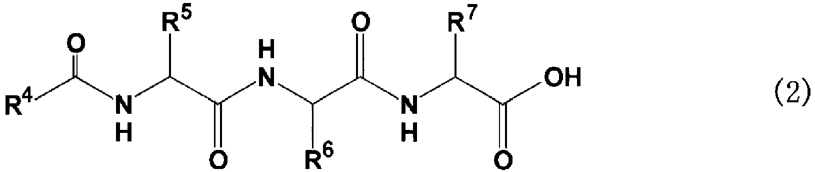 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); 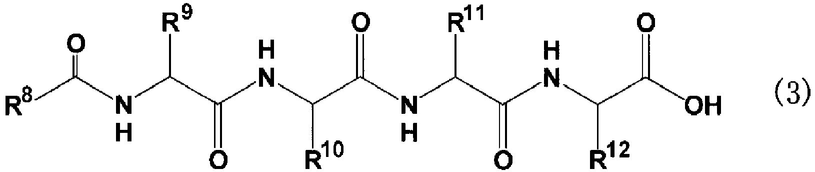 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring); or
pharmacologically usable salts thereof.

13. The aqueous composition according to claim 12, which further comprises at least one fatty acid.

14. The aqueous composition according to claim 12 or 13, which further comprises at least one polyhydric alcohol different from the 2-ethyl-1,3-hexanediol.

15. The aqueous composition according to any one of claims 12 to 14, which is a premix for preparing a solid base material for external use on skin.

16. A method for producing the solid base material for external use on skin according to claim 1, comprising:
a heating step of heating the aqueous composition according to any one of claims 12 to 15 to a temperature not lower than room temperature and lower than 100°C;
a mixing step of adding the heated aqueous composition to an aqueous phase heated to a temperature not lower than room temperature and lower than 100°C, followed by mixing, or adding an aqueous phase heated to a temperature not lower than room temperature and lower than 100°C to the heated aqueous composition, followed by mixing; and
a cooling step of cooling the resulting mixture to form a gel.

17. The method according to claim 16, wherein the aqueous phase comprises at least one oleaginous base.

18. The method according to claim 16 or 17, wherein the aqueous phase comprises at least one polyhydric alcohol different from the 2-ethyl-1,3-hexanediol.

19. The method according to claim 16, wherein the aqueous phase comprises at least one polymeric emulsifier.

## Patentansprüche

1. Festes Basismaterial zur äußerlichen Anwendung auf der Haut, umfassend:
ein Tensid, das aus einem oder mehreren Verbindungen, ausgewählt aus der Gruppe bestehend aus Ethylenglykolalkylethern, Phospholipiden, Polyglycerinfettsäureestern und Polyoxyethylenpolyoxypropylenalkylethern ausgewählt wird;
Wasser;
2-Ethyl-1,3-hexandiol; und
eine Lipidpeptidverbindung, umfassend mindestens eine der Verbindungen der Formeln (1) bis (3) oder ein pharmakologisch akzeptables Salz davon 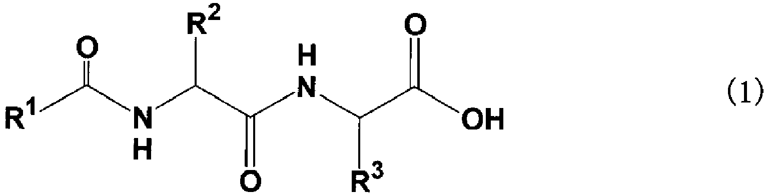
worin R¹ eine aliphatische C₉₋₂₃-Gruppe ist; R² ein Wasserstoffatom oder eine C₁₋₄-Alkylgruppe, die optional eine verzweigte C₁- oder C₂-Kette aufweist, ist; und worin R³ eine - (CH₂)ₙ-X-Gruppe ist, worin n eine Zahl von 1 ist bis 4 ist und X eine Aminogruppe, Guanidinogruppe, -CONH₂-Gruppe oder eine 5- oder 6-gliedrige Ringgruppe, die optional ein bis drei Stickstoffatome aufweist, oder eine kondensierte heterocyclische Gruppe, die aus dem 5-gliedrigen Ring und dem 6-gliedrigen Ring besteht, ist; 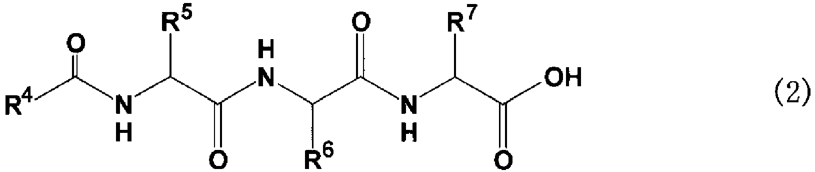 worin R⁴ eine aliphatische C₉₋₂₃-Gruppe ist; und worin R⁵ bis R⁷ jeweils unabhängig voneinander ein Wasserstoffatom, eine C₁₋₄-Alkylgruppe, die optional eine verzweigte C₁- oder C₂-Kette aufweist, oder eine -(CH₂)ₙ-X-Gruppe, worin n eine Zahl von 1 bis 4 ist, und X eine Aminogruppe, Guanidinogruppe, -CONH₂-Gruppe oder eine 5- oder 6-gliedrige Ringgruppe, die optional ein bis drei Stickstoffatome aufweist, oder eine kondensierte heterocyclische Gruppe, die aus dem 5-gliedrigen Ring und dem 6-gliedrigen Ring besteht, ist, sind; 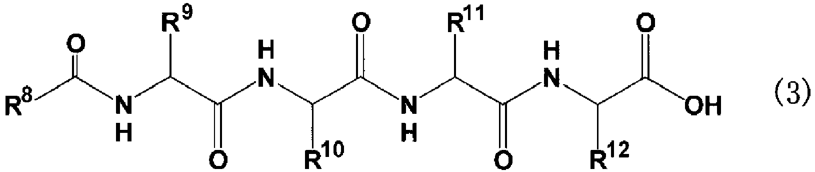 worin R⁸ eine aliphatische C₉₋₂₃-Gruppe ist; und R⁹ bis R¹² jeweils unabhängig voneinander ein Wasserstoffatom, eine C₁₋₄-Alkylgruppe, die optional eine verzweigte C₁- oder C₂-Kette aufweist, oder eine -(CH₂)ₙ-X-Gruppe, worin n eine Zahl von 1 bis 4 ist, und X eine Aminogruppe, Guanidinogruppe, -CONH₂-Gruppe oder eine 5- oder 6-gliedrige Ringgruppe, die optional ein bis drei Stickstoffatome aufweist, oder eine kondensierte heterocyclische Gruppe, die aus dem 5-gliedrigen Ring und dem 6-gliedrigen Ring besteht, ist, sind.

2. Festes Basismaterial zur äußerlichen Anwendung auf der Haut gemäß Anspruch 1, welches ferner mindestens eine Fettsäure enthält.

3. Festes Basismaterial zur äußerlichen Anwendung auf der Haut gemäß Anspruch 1 oder 2, welches ferner mindestens eine ölige Base enthält.

4. Festes Basismaterial zur äußerlichen Anwendung auf der Haut gemäß mindestens einem der Ansprüche 1 bis 3, welches ferner mindestens eine organische Säure, die sich von der Fettsäure unterscheidet, enthält.

5. Festes Basismaterial zur äußerlichen Anwendung auf der Haut gemäß mindestens einem der Ansprüche 1 bis 4, welches ferner mindestens einen mehrwertigen Alkohol, der sich von 2-Ethyl-1,3-hexandiol unterscheidet, enthält.

6. Festes Basismaterial zur äußerlichen Anwendung auf der Haut gemäß mindestens einem der Ansprüche 1 bis 5, welches ferner mindestens einen polymeren Emulgator enthält.

7. Festes Basismaterial zur äußerlichen Anwendung auf der Haut gemäß mindestens einem der Ansprüche 2 bis 6, wobei die Fettsäure Stearinsäure ist.

8. Festes Basismaterial zur äußerlichen Anwendung auf der Haut gemäß mindestens einem der Ansprüche 5 bis 7, wobei der mehrwertige Alkohol Glycerin, Propylenglycol oder Polyethylenglycol ist.

9. Festes Basismaterial zur äußerlichen Anwendung auf der Haut gemäß mindestens einem der Ansprüche 6 bis 8, wobei der polymere Emulgator mindestens eine Polymerverbindung, ausgewählt aus der Gruppe bestehend aus Pfropfpolymerverbindungen, die jeweils eine hydrophile Hauptkette und eine darauf aufgepfropfte hydrophobe Einheit aufweisen, und Blockpolymerverbindungen, die jeweils aus hydrophoben und hydrophilen Struktureinheiten zusammengesetzt sind, ist.

10. Festes Basismaterial zur äußerlichen Anwendung auf der Haut gemäß mindestens einem der Ansprüche 1 bis 9, welches für Kosmetika oder Pharmazeutika verwendet wird.

11. Festes Basismaterial zur äußerlichen Anwendung auf der Haut gemäß mindestens einem der Ansprüche 1 bis 10, welches stiftförmig ist.

12. Wässrige Zusammensetzung, umfassend:
ein Tensid, das aus einem oder mehreren Verbindungen, ausgewählt aus der Gruppe bestehend aus Ethylenglykolalkylethern, Phospholipiden, Polyglycerinfettsäureestern und Polyoxyethylenpolyoxypropylenalkylethern, ausgewählt wird;
Wasser;
2-Ethyl-1,3-hexandiol; und
eine Lipidpeptidverbindung, umfassend mindestens eine der Verbindungen der Formeln (1) bis (3) oder ein pharmakologisch akzeptables Salz davon 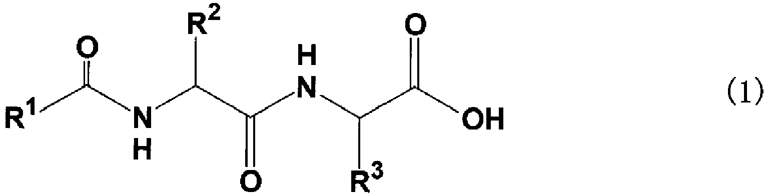
worin R¹ eine aliphatische C₉₋₂₃-Gruppe ist; R² ein Wasserstoffatom oder eine C₁₋₄-Alkylgruppe, die optional eine verzweigte C₁- oder C₂-Kette aufweist, ist; und worin R³ eine - (CH₂)ₙ-X-Gruppe ist, worin n eine Zahl von 1 ist bis 4 ist und X eine Aminogruppe, Guanidinogruppe, -CONH₂-Gruppe oder eine 5- oder 6-gliedrige Ringgruppe, die optional ein bis drei Stickstoffatome aufweist, oder eine kondensierte heterocyclische Gruppe, die aus dem 5-gliedrigen Ring und dem 6-gliedrigen Ring besteht, ist; 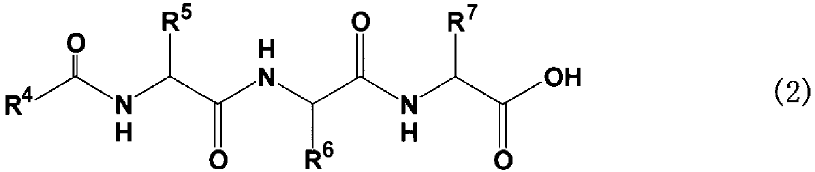 worin R⁴ eine aliphatische C₉₋₂₃-Gruppe ist; und worin R⁵ bis R⁷ jeweils unabhängig voneinander ein Wasserstoffatom, eine C₁₋₄-Alkylgruppe, die optional eine verzweigte C₁- oder C₂-Kette aufweist, oder eine -(CH₂)ₙ-X-Gruppe, worin n eine Zahl von 1 bis 4 ist, und X eine Aminogruppe, Guanidinogruppe, -CONH₂-Gruppe oder eine 5- oder 6-gliedrige Ringgruppe, die optional ein bis drei Stickstoffatome aufweist, oder eine kondensierte heterocyclische Gruppe, die aus dem 5-gliedrigen Ring und dem 6-gliedrigen Ring besteht, ist, sind; 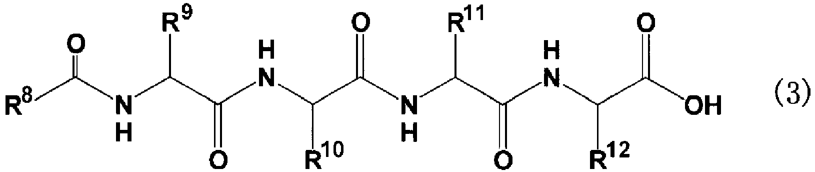 worin R⁸ eine aliphatische C₉₋₂₃-Gruppe ist; und R⁹ bis R¹² jeweils unabhängig voneinander ein Wasserstoffatom, eine C₁₋₄-Alkylgruppe, die optional eine verzweigte C₁- oder C₂-Kette aufweist, oder eine -(CH₂)ₙ-X-Gruppe, worin n eine Zahl von 1 bis 4 ist, und X eine Aminogruppe, Guanidinogruppe, -CONH₂-Gruppe oder eine 5- oder 6-gliedrige Ringgruppe, die optional ein bis drei Stickstoffatome aufweist, oder eine kondensierte heterocyclische Gruppe, die aus dem 5-gliedrigen Ring und dem 6-gliedrigen Ring besteht, ist, sind.

13. Wässrige Zusammensetzung gemäß Anspruch 12, welche ferner mindestens eine Fettsäure enthält.

14. Wässrige Zusammensetzung gemäß Anspruch 12 oder 13, welche ferner mindestens einen mehrwertigen Alkohol, der sich von 2-Ethyl-1,3-hexandiol unterscheidet, enthält.

15. Wässrige Zusammensetzung gemäß mindestens einem der Ansprüche 12 bis 14, welche als Vormischung für die Herstellung eines festen Basismaterials zur äußerlichen Anwendung auf der Haut dient.

16. Verfahren zur Herstellung des festen Basismaterials zur äußerlichen Anwendung auf der Haut gemäß Anspruch 1, umfassend
einen Erwärmungsschritt umfassend das Erwärmen der wässrigen Zusammensetzung gemäß mindestens einem der Ansprüche 12 bis 15 auf eine Temperatur, die nicht niedriger als Raumtemperatur und niedriger als 100°C ist;
einen Mischschritt umfassend das Hinzufügen der erwärmten wässrigen Zusammensetzung zu einer wässrigen Phase, die auf eine Temperatur von nicht niedriger als Raumtemperatur und niedriger als 100°C erwärmt wurde, gefolgt von Mischen, oder das Hinzufügen einer wässrigen Phase, die auf eine Temperatur von nicht niedriger als Raumtemperatur und niedriger als 100°C erhitzt wurde, zu der erwärmten wässrigen Zusammensetzung, gefolgt von Mischen; und
einen Abkühlungsschritt umfassend das Abkühlen der resultierenden Mischung zum Bilden eines Gels.

17. Verfahren gemäß Anspruch 16, wobei die wässrige Phase mindestens eine ölige Base enthält.

18. Verfahren gemäß Anspruch 16 oder 17, wobei die wässrige Phase mindestens einen mehrwertigen Alkohol, der sich von 2-Ethyl-1,3-hexandiol unterscheidet, enthält.

19. Verfahren gemäß Anspruch 16, wobei die wässrige Phase mindestens einen polymeren Emulgator enthält.

## Revendications

1. Matériau de base solide à usage externe sur la peau comprenant :
un tensioactif qui est un ou plusieurs composés choisis parmi le groupe constitué d'éthers alkyliques d'éthylène glycol, de phospholipides, d'esters d'acide gras de polyglycérine, et d'éthers alkyliques de polyoxyéthylène polyoxypropylène ;
de l'eau ;
un 2-éthyl-1,3-hexanediol ; et
un composé peptide lipidique comprenant au moins l'un des composés de formules (1) à (3) : 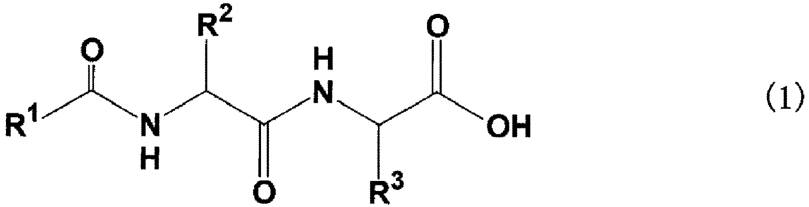
(où R¹ est un groupe aliphatique en C₉₋₂₃ ; R² est un atome d'hydrogène ou un groupe alkyle en C₁₋₄ ayant facultativement une chaîne ramifiée en C₁ ou C₂ ; et R³ est un groupe -(CH₂)ₙ-X, où n est un nombre compris entre 1 et 4, et X est un groupe amino, un groupe guanidino, un groupe -CONH₂, ou un groupe cyclique à 5 chaînons ou un groupe cyclique à 6 chaînons ayant facultativement un à trois atomes d'azote ou un groupe hétérocyclique fusionné composé du cycle à 5 chaînons et du cycle à 6 chaînons) ; 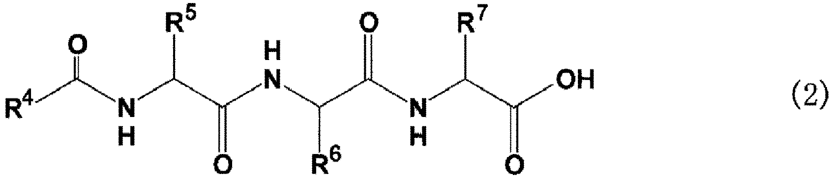 (où R⁴ est un groupe aliphatique en C₉₋₂₃ ; et R⁵ à R⁷ sont chacun indépendamment un atome d'hydrogène, un groupe alkyle en C₁₋₄ ayant facultativement une chaîne ramifiée en C₁ ou C₂ ; ou un groupe -(CH₂)ₙ-X, où n est un nombre compris entre 1 et 4, et X est un groupe amino, un groupe guanidino, un groupe -CONH₂, ou un groupe cyclique à 5 chaînons ou un groupe cyclique à 6 chaînons ayant facultativement un à trois atomes d'azote ou un groupe hétérocyclique fusionné composé du cycle à 5 chaînons et du cycle à 6 chaînons) ; 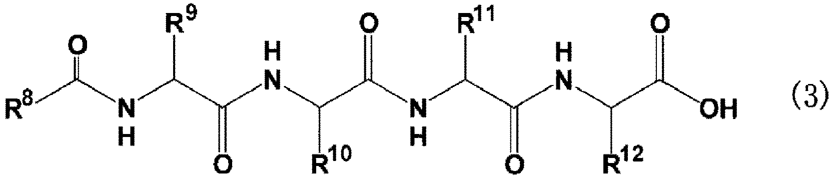 (où R⁸ est un groupe aliphatique en C₉₋₂₃ ; et R⁹ à R¹² sont chacun indépendamment un atome d'hydrogène, un groupe alkyle en C₁₋₄ ayant facultativement une chaîne ramifiée en C₁ ou C₂ ; ou un groupe -(CH₂)ₙ-X, où n est un nombre compris entre 1 et 4, et X est un groupe amino, un groupe guanidino, un groupe -CONH₂, ou un groupe cyclique à 5 chaînons ou un groupe cyclique à 6 chaînons ayant facultativement un à trois atomes d'azote ou un groupe hétérocyclique fusionné composé du cycle à 5 chaînons et du cycle à 6 chaînons) ; ou
des sels pharmacologiquement utilisables de ceux-ci.

2. Matériau de base solide à usage externe sur la peau selon la revendication 1, qui comprend en outre au moins un acide gras.

3. Matériau de base solide à usage externe sur la peau selon la revendication 1 ou 2, qui comprend en outre au moins une base oléagineuse.

4. Matériau de base solide à usage externe sur la peau selon l'une quelconque des revendications 1 à 3, qui comprend en outre au moins un acide organique différent de l'acide gras.

5. Matériau de base solide à usage externe sur la peau selon l'une quelconque des revendications 1 à 4, qui comprend en outre au moins un alcool polyhydrique différent du 2-éthyl-1,3-hexanediol.

6. Matériau de base solide à usage externe sur la peau selon l'une quelconque des revendications 1 à 5, qui comprend en outre au moins un émulsifiant polymère.

7. Matériau de base solide à usage externe sur la peau selon l'une quelconque des revendications 2 à 6, dans lequel l'acide gras est un acide stéarique.

8. Matériau de base solide à usage externe sur la peau selon l'une quelconque des revendications 5 à 7, dans lequel l'alcool polyhydrique est une glycérine, du propylène glycol ou du polyéthylène glycol.

9. Matériau de base solide à usage externe sur la peau selon l'une quelconque des revendications 6 à 8, dans lequel l'émulsifiant polymère est au moins un composé polymère choisi parmi le groupe constitué de composés polymères greffés ayant chacun une chaîne principale hydrophile et un fragment hydrophobe greffé sur celle-ci et de composés polymères séquencés composés chacun d'unités structurelles hydrophobes et hydrophiles.

10. Matériau de base solide à usage externe sur la peau selon l'une quelconque des revendications 1 à 9, qui est utilisé pour des cosmétiques ou produits pharmaceutiques.

11. Matériau de base solide à usage externe sur la peau selon l'une quelconque des revendications 1 à 10, qui est en forme de bâtonnet.

12. Composition aqueuse comprenant :
un tensioactif qui est un ou plusieurs composés choisis parmi le groupe constitué d'éthers alkyliques d'éthylène glycol, de phospholipides, d'esters d'acide gras de polyglycérine, et d'éthers alkyliques de polyoxyéthylène polyoxypropylène ;
de l'eau ;
un 2-éthyl-1,3-hexanediol ; et
un composé peptide lipidique comprenant au moins l'un des composés de formules (1) à (3) : 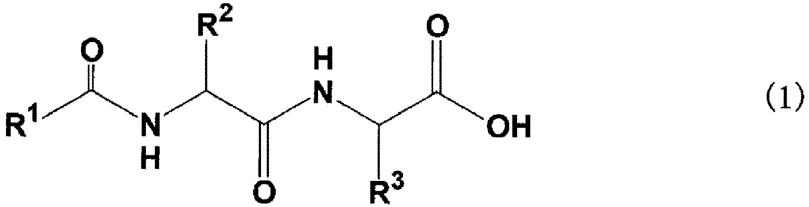
(où R¹ est un groupe aliphatique en C₉₋₂₃ ; R² est un atome d'hydrogène ou un groupe alkyle en C₁₋₄ ayant facultativement une chaîne ramifiée en C₁ ou C₂ ; et R³ est un groupe -(CH₂)ₙ-X, où n est un nombre compris entre 1 et 4, et X est un groupe amino, un groupe guanidino, un groupe -CONH₂, ou un groupe cyclique à 5 chaînons ou un groupe cyclique à 6 chaînons ayant facultativement un à trois atomes d'azote ou un groupe hétérocyclique fusionné composé du cycle à 5 chaînons et du cycle à 6 chaînons) ; 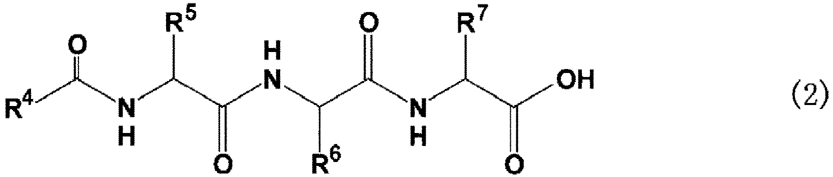 (où R⁴ est un groupe aliphatique en C₉₋₂₃ ; et R⁵ à R⁷ sont chacun indépendamment un atome d'hydrogène, un groupe alkyle en C₁₋₄ ayant facultativement une chaîne ramifiée en C₁ ou C₂ ; ou un groupe -(CH₂)ₙ-X, où n est un nombre compris entre 1 et 4, et X est un groupe amino, un groupe guanidino, un groupe -CONH₂, ou un groupe cyclique à 5 chaînons ou un groupe cyclique à 6 chaînons ayant facultativement un à trois atomes d'azote ou un groupe hétérocyclique fusionné composé du cycle à 5 chaînons et du cycle à 6 chaînons) ; 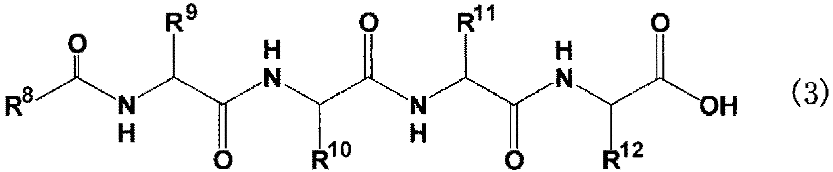 (où R⁸ est un groupe aliphatique en C₉₋₂₃ ; et R⁹ à R¹² sont chacun indépendamment un atome d'hydrogène, un groupe alkyle en C₁₋₄ ayant facultativement une chaîne ramifiée en C₁ ou C₂ ; ou un groupe -(CH₂)ₙ-X, où n est un nombre compris entre 1 et 4, et X est un groupe amino, un groupe guanidino, un groupe -CONH₂, ou un groupe cyclique à 5 chaînons ou un groupe cyclique à 6 chaînons ayant facultativement un à trois atomes d'azote ou un groupe hétérocyclique fusionné composé du cycle à 5 chaînons et du cycle à 6 chaînons) ; ou
des sels pharmacologiquement utilisables de ceux-ci.

13. Composition aqueuse selon la revendication 12, qui comprend en outre au moins un acide gras.

14. Composition aqueuse selon la revendication 12 ou 13, qui comprend en outre au moins un alcool polyhydrique différent du 2-éthyl-1,3-hexanediol.

15. Composition aqueuse selon l'une quelconque des revendications 12 à 14, qui est un pré-mélange pour préparer un matériau de base solide pour un usage externe sur la peau.

16. Procédé de production du matériau de base solide pour usage externe sur la peau selon la revendication 1, comprenant :
une étape de chauffage consistant à chauffer la composition aqueuse selon l'une quelconque des revendications 12 à 15 à une température qui n'est pas inférieure à la température ambiante et inférieure à 100 °C ;
une étape de mélange consistant à ajouter la composition aqueuse chauffée à une phase aqueuse chauffée à une température qui n'est pas inférieure à la température ambiante et inférieure à 100 °C, suivie d'un mélange, ou d'un ajout d'une phase aqueuse chauffée à une température qui n'est pas inférieure à la température ambiante et inférieure à 100 °C à la composition aqueuse chauffée, suivi d'un mélange ; et
une étape de refroidissement consistant à refroidir le mélange résultant pour former un gel.

17. Procédé selon la revendication 16, dans lequel la phase aqueuse comprend au moins une base oléagineuse.

18. Procédé selon la revendication 16 ou 17, dans lequel la phase aqueuse comprend au moins un alcool polyhydrique différent du 2-éthyl-1,3-hexanediol.

19. Procédé selon la revendication 16, dans lequel la phase aqueuse comprend au moins un émulsifiant polymère.
